(19) Europäisches Patentamt / European Patent Office / Office européen des brevets

(11) **EP 4 265 111 A1**

(12) **EUROPEAN PATENT APPLICATION**
published in accordance with Art. 153(4) EPC

(43) Date of publication:
**25.10.2023 Bulletin 2023/43**

(21) Application number: **20964452.5**

(22) Date of filing: **08.12.2020**

(51) International Patent Classification (IPC):
**A01N 63/22** (2020.01)   **C12N 1/20** (2006.01)
**C12R 1/07** (2006.01)   **C05G 3/00** (2020.01)
**C05F 11/08** (2006.01)

(52) Cooperative Patent Classification (CPC):
**A01N 63/22; C05F 11/08; C05G 3/00; C12N 1/20;**
C12R 2001/07

(86) International application number:
**PCT/CL2020/050169**

(87) International publication number:
**WO 2022/120503 (16.06.2022 Gazette 2022/24)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Blanes SpA**
**Santiago, 7570170 (CL)**

(72) Inventors:
- **MONTENEGRO MELGAR, Edgar Armando**
  **Ciudad de Guatemala, Guatemala 01011 (GT)**
- **PINEDA MIJANGOS, Wilson Guillermo**
  **Ciudad de Guatemala, Guatemala 01011 (GT)**

- **DARDÓN FRESSE DE ÁVALOS, Elena María**
  **Ciudad de Guatemala, Guatemala 01011 (GT)**
- **RAMAZZINI SANTOS, Héctor Ronaldo**
  **Ciudad de Guatemala, Guatemala 01011 (GT)**
- **GARCÍA-GALLONT, Ignacio Viteri**
  **Ciudad de Guatemala, Guatemala 01011 (GT)**
- **VITERI ARRIOLA, Francisco**
  **Ciudad de Guatemala, Guatemala 01011 (GT)**
- **DELGADO HERNÁNDEZ, Verónica Melissa**
  **Alta Vista, La Florida  Santiago (CL)**

(74) Representative: **Elzaburu S.L.P.**
**Edificio Torre de Cristal**
**Paseo de la Castellana 259 C, planta 28**
**28046 Madrid (ES)**

(54) **AGRICULTURAL FORMULATION COMPRISING AT LEAST ONE BACTERIAL STRAIN B. SAFENSIS RGM 2450, AND/OR A BACTERIAL STRAIN B. SIAMENSIS RGM 2529 AND AGRICULTURAL EXCIPIENTS; USE OF THE FORMULATION AND METHOD FOR STIMULATING GROWTH AND/OR INCREASING CROP YIELDS AND/OR PROTECTING CROPS AGAINST DISEASES AND PESTS**

(57)    The invention relates to an agricultural formulation for stimulating growth and/or increasing crop yields and/or protecting crops against diseases and pests, comprising at least one bacterial strain of B. safensis having deposit number RGM 2450 and/or a bacterial strain of B. siamensis having deposit number RGM 2529 and excipients for agricultural use. The composition serves as a crop bioprotector and/or biocontroller. The invention also relates to a method for stimulating growth and/or increasing crop yields and/or protecting crops against diseases and pests.

EP 4 265 111 A1

**Description**

**Field of Invention**

The present invention is directed at agricultural formulations containing bacteria or mixtures thereof, which exhibit culture growth enhancer and biological pest control characteristics. The present invention focuses on the area of biotechnology, particularly on the development of agricultural products that enhance crop growth and prevent and control culture pathogen infection using formulations containing one or a mixture of bacteria with these characteristics.

**Description of the State of the Art**

Agricultural production requires sustainable measures that allow for the reduction and good use of water, fertilizers and pesticides, but also allow for maintaining soil quality, strengthening crop growth, and preventing pests, the latter causing large economic losses. Charitable microorganism-based agribusiness inputs have emerged in response to demand from global markets for high-quality, environmentally-friendly, traceable and safe food (Altier *et al.,* 2012).

Biological agribusiness products are formulated with microorganisms (e.g., bacteria, fungi, viruses) or with active compounds derived from microorganisms or plants. All of these products focus on improving productivity, quality, plant health, and soil biological characteristics (Altier et al., 2012). In the case of products containing microorganisms, these are selected for their ability to promote plant growth directly by facilitating the absorption of nutrients by the plant (biofertilizers) or indirectly by contributing to the health management of pest diseases with economic impact (Biocontrollers). Of note, many of these strains that are used for these purposes may exhibit fertilizer and/or biocontroller characteristics (Altier et al., 2012).

The microorganisms that help plants grow are called PGPR (Plant Growth Promoting Rhizobacteria). Soil is an ecosystem that has a variety of beneficial microorganisms. The soil fraction that presents this type of microorganism is found where there is the greatest presence of roots (rhizosphere) (Chollo et al., 2012). In the rhizosphere, bacterial consortiums that promote plant growth can live together directly and indirectly (Mayak et al. 1999), colonize the roots or their nearest environment, and promote plant growth using atmospheric nitrogen binding mechanisms, solubilization of insoluble phosphates, and secretion of hormones and enzymes (Glick et al., 1994; Chollo et al., 2012). These bacteria can be classified into three main groups: a) those that colonize plant tissue and form nodules (symbiotics), b) those that are housed in internal structures of the plant (endophytic), and c) those that are located near the plant's root system (Chollo et al., 2012).

Multiple studies have published that PGPRs are associated with important crops such as *Oryiza sativa, Triticum spp., Sorghum spp., Sacharum officinarum, Zea mays,* and grasses. Among the most commonly referenced PGPRs are *Azospirillum sp., Bacillus sp., Rhizobium sp., Burkholderia sp., Enterobacter sp., Azotobacter sp., Erwinia sp., Herbaspirillum sp., Klebsiella sp., Pseudomonas sp., and Xanthomonas sp.* (Chollo et al., 2012).

Among the PGPR bacteria mentioned in the previous paragraph are those belonging to the genus *Bacillus.* These gram-positive bacteria are ubiquitous, i.e., they can live in different ecological niches, including soil (Pignatelli et al., 2009). *Bacillus* bacteria that live in soil can have PGPR characteristics. For example, these bacteria have the ability to bind atmospheric nitrogen ($N_2$). Nitrogenase-like enzymes belonging to this bacteria reduce N2 to ammonia (NH3), the latter being absorbed by the plant. In addition, these bacteria play an important role in the solubilization of phosphates (P) so that this compound can be available to plants by using enzymes such as phosphatase, phytase, hydrolases, and others (Hayat et al., 2010). With regard to the solubilization of components performed by these bacteria, potassium (K) solubilization is also presented. This compound is necessary for the activation of various enzymes from plants and animals intended to participate in energy metabolism, such as starch synthesis, nitrate reduction, sugar degradation and, one of the most important functions, photosynthesis (Etesami et al., 2017).

In addition to the characteristics of bacteria of the genus *Bacillus* in the binding and solubilization of compounds that promote plant growth, the secretion of compounds with antimicrobial activity also occurs. These antimicrobial compounds are secreted by bacteria that act as antagonists against other pathogenic species. These compounds are molecules, particularly non-ribosomal synthesis peptides and polypeptides composed of polymethides, bacteriokines and siderophores, as well as volatile compounds. They generally have a broad spectrum of action (bacteria, fungi) and *Bacillus* strains exhibit predominant biological control capacity by secreting these molecules, inhibiting pathogen growth in plants.

*- Bacillus safensis*

Described as a plant growth promoter and pathogenic control agent, *Bacillus safensis* is a species that has the ability to become an endophyte and inhabit the rhizosphere of plants, promoting plant growth. To enhance plant growth, *B. safensis* solubilizes soil phosphate (P) and siderophore production, indole-3-acetic acid, and 1-aminocyclopropane-1-carboxylate deaminase (Yadav et al., 2011; Chakraborty et al., 2013; Kavamura et al., 2013). In addition, it has been reported that this bacteria can act as a biocontroller, presenting antifungal, antibacterial and antiviral effects in various crops, individually or in the company of other bacteria (Sun et al., 2014).

- *Bacillus siamensis*

This bacteria of the genus *Bacillus* was isolated in 2010 from a Thai crab, calling this strain *B. siamensis* KCTC 13613. This strain has been shown to have the ability to significantly inhibit the growth of mycelium from the pathogenic fungi of *Rhizoctonia solani* and *Botrytis cinerea* plants, in addition to exhibiting antimicrobial activity on the gram-positive bacteria *Micrococcus luteus* (Jeong et al., 2012). On the other hand, it has been observed that *B. siamensis* can significantly increase the growth of seedlings of *Arabidopsis thaliana* without physical contact with seedlings, suggesting that volatile substances produced by these bacteria could promote plant growth (Jeong et al., 2012).

Various state-of-the-art documents have published on the qualities of microorganisms, such as bacteria, to enhance plant growth and to act as biological controllers on other pathogens affecting plants. For example, the document by Haeyoung et al., published in 2012, describes a new species of halophilic bacteria, belonging to the genus *Bacillus,* which is able to produce antimicrobial compounds against plant pathogens (particularly fungi such as *Rhizoctonia solani* and *Botrytis cinerea)* and promote the growth of these by emission of volatile compounds. The scientific document published by Wu and colleagues in 2015 presents a review of biocontrol formulations that are generated from plant growth-promoting bacteria, which have been shown to be an alternative for the control of pathogens in crops, in addition to enhancing plant growth.

Bacteria of the genus *Bacillus* have been described as having characteristics that allow them to live with plants, particularly in the rhizosphere, and promote plant growth. *Bacillus* bacteria have also been described as producing antimicrobial compounds so that they can control the growth of other pathogens in plants. These characteristics have been described in documents such as that submitted by Borris, R., in 2011.

With respect to the invention, the documents of Agbaje et al. (2015) and Haeyoung et al. (2012) have described the characteristics of *B. siamensis* and *B. safaensis* in promoting the growth of plants as their antagonism in the growth of pathogens.

With respect to the invention, patent documents are presented that reveal the use of microorganisms of the genus *Bacillus,* particularly *B. siamensis* and *B safensis,* as growth-enhancing agents in plants and biocontrollers. Such is the case with patent documents CN 109468243 and KR 1020190061210, which present the use of *B siamensis;* specifically, the first document discloses the use of *B. siamensis* as a biocontrolling agent, since it presents significant antagonism against pathogens. In the specific case of this document, the inhibition of growth where they cause rotting of stems and spots on leaves is presented. Meanwhile, the second document discloses the use of this bacteria since it has nitrogen reduction characteristics, so it is therefore indicated that it can be used to enhance plant growth or for water treatment.

Documents CN109456915 and CN108330092 disclose the use of *B. safensis* bacteria as a plant growth enhancing agent. These documents describe the characteristics of this bacteria, such as phosphorus (P) degradation in soil. On the other hand, documents KR1020140079201, CN108865934, CN108330092, and CN106119146 describe the use of this bacteria and/or its cultures as antagonists for the growth of plant pathogens, such as *B. kinérea.*

**Detailed Description of the Invention**

The present invention corresponds to an agricultural formulation, which is composed of one or two bacterial strains that exhibit plant growth promoting characteristics (PGPRs) and bioprotectant and biocontroller characteristics. The formulation may comprise the bacteria individually or a mixture of the strains *Bacillus safensis* RGM 2450 and *Bacillus siamensis* RGM 2529. Both bacteria are defined according to their registration or deposit number in the IDA microbial bank in Chilean genetic microbial sources in INIA, Quilamapu, Chile.

The bacteria of the present invention are used by way of a formulation and have as their characteristic a biostimulant, bioprotective and biocontrolling function.

Another aspect of the present invention relates to a biofertilizer that enhances crop yield, comprising mixing growth promoting bacteria in agriculturally acceptable plants and excipients.

Another aspect of the present invention protects two processes that optimize agricultural crops. In the first instance, the present invention protects the well-being of plants. In this regard, the present invention aims to increase the availability of elements that are considered nutrients for plants, such as nitrogen ($N_2$), potassium (K) and phosphorus (P), by enhancing their growth. In the present invention, increased nutrient availability for plants is generated using naturally occurring soil bacteria, which present the ability to solubilize these compounds (K and P) and bind nitrogen ($N_2$).

Second, the safety of the crops is protected in the present invention by protection against pathogens. In the present invention, this protection is given as the bacteria used have the ability to release substances or molecules that are harmful to pathogenic organisms. Considering both characteristics (enhancing plant growth and protecting pathogens), the present invention manages to take advantage of the biological mechanisms of these bacteria and consequently reduce or completely eliminate the application of agrochemicals or fertilizers necessary in the soil, reducing the environmental and human impact. In particular, the *Bacillus safensis* RGM 2450 and *Bacillus siamensis* RGM 2529 bacteria would produce antimicrobial compounds against a broad spectrum of bacteria and fungi. For *B. siamensis* RGM 2529, it has been shown that this strain would potentially produce antimicrobial compounds with antibacterial effect on *S. aureus ATCC 25923, M. luteus CMCC 28001, B. pumilus CMCC 63202, B. cereus ATCC 14579, B. subtilis ATCC 168, Listeria monocytogenes CICC 21662, Enterococcus faecalis ATCC 29212* and *P. fluorescens ATCC 49642, B. thuringiensis, E. coli, Klebsiella pneumoniae, M. xanthus, P. vulgaris, Serratia marcescens, S. aureus, L. pneumophila, L. monocytogenes, P. syringae, A. solanacearum, Xanthomonas axonopodis pv., Glycines, Brevibacillus brevis, Bacillus cereus, Bacillus licheniformis, Bacillus megaterium, B. pumilus, Bacillus sphaericus, B. subtilis, Clavibacter michiganensis, Micrococcus luteus, Paenibacillus granivorans,* and *Paenibacillus polymyxa.* As for fungi, these are included within the scope of the bioprotective effect of the formulation: *Coriolopsis spp., Fusarium sp, Pseudoxylaria sp., Trichoderma sp., Umbelopsis sp., F. oxysporum f. sp., Capsici, A. niger, B. cinerea, F. solani, Monilia fructigena, Pennicilium expansum, P. italicum, R. solani, Alternaria alternata, A. solani, Aspergillus flavus, Botryosphaerica ribis, C. albicans, Cryphonectria parasítica, Colletotrichum acutatum, Colletotrichum gloesporioides, Didymella bryoniae, F. graminearum, F. oxysporum, Ustilago maydis, Monilinia fructicola, Penicillium expansum, Phomopsis gossypii, Phytophthora capsici, Pyricularia grisea, R. solani, Sclerotium rolfsii, S. sclerotiorum, Alternaría solani, Botrytis cinérea, Fusarium graminearum, Fusarium sambucinum, Fusarium oxysporum, Podosphaera fusca, Pythium sulcatum, Pythium ultimum, Rhizoctonia solani, Rhizopus* sp., and *Sclerotinia sclerotiorum. B. safensis* RGM 2450 would produce antimicrobial compounds against *Clavibacter michiganense subsp. sepedonicum, Erwinia amylovora, Escherichia coli, Salmonella typhi, Staphylococcus aureus, Streptococcus pyogenes, Brevibacillus brevis, Bacillus cereus, Bacillus licheniformis, Bacillus megaterium, Bacillus pumilus, Bacillus sphaericus, B. subtilis, Micrococcus luteus,* and *Paenibacillus granivorans.* It could also have an antifungal effect on fungi such as *Candida albicans, Microcystis aeruginosa, Phytophthora infestans, S. cerevisiae, Aspergillus fumigatus, Candida albicans,* and *F. oxysporum f. sp. Capsici.*

Both strains can also secrete enzymes such as proteases, serinproteases, among others, for which their antibacterial and antifungal effect has been described. Thus, as part of the scope of the invention, the bioprotective effect of strains is included independently and collectively in the formulation against a wide variety of fungi and phytopathogenic bacteria.

In a third aspect, the disclosed formulation and bacteria exhibit a biocontrolling effect of phytopathogens, i.e., it is intended to decrease the growth of the pathogen population, in this case in crops and plants. The bacteria and the formulation are shown to have antibacterial effect against *Fusarum* sp., *Phytophthora* sp., *Collectotrichum* sp., and *Botrytis* sp.

The results obtained evaluated in *Fusarum* sp. show that the RGM 2450 and RGM 2529 strains caused a growth inhibition of 24% and 4% by direct contact, and 53% and 41% by antibiotic assay, respectively. On the other hand, the RGM 2450 strain caused 45% inhibition and 41% antibiosis when in direct contact with *Phytophthora* sp., and the RGM 2529 strain showed 80% inhibition and 52% antibiosis. For both *Fusarum* sp. and *Phytophthora* sp. both bacterias inhibit the production of volatile compounds.

For *Collectotrichum* sp., the RGM 2450 strain inhibited 43% and 53% for the direct contact and antibiosis assays, respectively; for RGM 2529, a decrease in the pathogen growth halo of 78% was observed by direct contact, and of 70% by antibiosis.

For *Botrytis,* the assays demonstrate that the RGM 2450 and RGM 2529 strains inhibit the growth of this phytopathogen significantly more than the commercial reference product devoid of its coformulants (bacteria *Bacillus subitillis* QST 713), achieving a 50% inhibition percentage both by using each RGM strain separately, and by applying the mixture of both.

In another aspect of the invention, the mixture of bacterial strains is combined to obtain a biofertilizer according to the principles of the present invention, wherein the bacterial strains are at preferred concentrations $3.0 \times 10^6$ - $4.0 \times 10^9$ CFU/g (RGM 2529) and $5.0 \times 10^6$ - $4 \times 10^9$ CFU/g (RGM 2450).

Another aspect of the invention relates to a biofertilizer comprising the mixture of bacteria and an agriculturally acceptable excipient that are intended to enhance the growth of the crop's plants.

In another aspect of the invention, the excipient of agricultural and/or agriculturally acceptable use comprises, but is not limited to: the use of wheat flour, corn starch, gelatin, potato starch, silicon dioxide, citric acid, bicarbonate, polysorbates like Tweens, lactose, soy lecithin, casein, carboxymethyl cellulose or cellulose gum, sucrose esters, mannitol, sorbitans, Pluronic F68, alginate, xanthan gum, PEG (polyethylene glycol), corn syrup, egg, milk, glycerol, fructose, pectins, mineral oil, ester gum, long-chain triglycerides.

In another aspect of the invention, a biocontroller comprises one organism that inhibits the growth of another, the latter being a pathogenic organism. In a particular aspect of the invention, the biocontroller comprises the mixture of the strains *Bacillus safensis* RGM 2450 and *Bacillus siamensis* RGM 2529.

In another aspect of the invention, the biofertilizer/biocontroller of the present invention may bear a pre-packaged soil, a seed coater, a powder, a granulate, a nebulizer, a suspension or a liquid, or any of the exposed encapsulated variants.

It is part of the specific scope of the invention that the strains *Bacillus safensis* RGM 2450 and *Bacillus siamensis* RGM 2529 are part of a formulation that can be administered as an extended release tablet, a wettable powder solid, as an effervescent tablet, as a resuspension, bacterial emulsion, or mixtures thereof. It is part of the scope of the invention to administer the formulation in conjunction with fertilizers. It is preferred to administer it with percentages of 0, 33, 66 and 100% fertilizer.

The different formulation presentation forms may be administered to the seed or at the base of the stem. In the case of seeds, they are impregnated with PBS buffer or similar and completely submerged in the formulation, preferring a concentration of $10^9$ CFU/mL of bacteria. For application at the base of the plant stem, a concentration of $10^8$ CFU/mL is preferred.

The biofertilizing/biocontroller formulation is effective for both plants used in the area of agriculture, as well as for obtaining food from animals and humans, for example, fruits and vegetables, and for plants that can be grown with the goal of simply being ornamental.

The formulation part of the present invention is focused on the use thereof in agriculture, which may comprise, but are not limited to, species of tomato (*Solanum lycopersicum*), banana (*Musa paradisiaca*), sugar cane (*Saccharum officinarum*), bell pepper (*Capsicum annuum*), papaya (*Carica papaya*), corn (*Zea mays*), watermelon (*Citrullus lanatus*), melon (*Cucumis melo*), avocado (*American Persea*), coffee (*Coffea arabica*), pea (*Pisum sativum*), bean (*Phaseolus vulgaris*), cane (*Cannabis sativa*), wheat (*Triticum spp*), rice (*Oryza sativa*), pineapple (*Ananas assus*), grape (*Vitis vinifera*), cherry (*Prunus cerasus*), apple (*Malus domestica*), strawberry (*Fragaria ananassa*), and lettuce (*Lactuca sativa*), among other crops.

The agricultural formulation described in the present invention has a method of action that targets the growing plants and pathogens that affect it, so direct contact of this product with humans could not cause any harm. On the other hand, the biological product of the present invention allows for a reduction of greenhouse gases since it decreases the use of traditional agrochemicals, representing an alternative for the replacement of the latter and allows compliance with environmental standards such as those established in the Organization for Economic Cooperation and Development (OECD).

It is also part of the scope of the invention, a method of promoting growth and/or increasing crop yields and/or protecting them against diseases and pests by administering or applying an effective amount of the described formulation. The method includes administration of the agricultural formulation in the form of resuspension, emulsion, effervescent tablet, extended-release tablet, or wettable powders.

As part of the method described for improving the well-being of plants and protecting them against disease, the formulation may be applied by sprinkling or direct immersion of the seed of the crop of interest and/or applying it to the base of the plants' stem. For seed immersion, a concentration of $10^9$ CFU/mL of bacteria is preferred. When applied to the base of the plant stem, a concentration of $10^8$ CFU/mL of bacteria is preferred.

The inventors have shown that, with the method described, when the formulation is applied to tomatoes, its productive capacity is increased by using the bacteria mixture and 66% complementary conventional fertilization. In particular, a significant increase in the cumulative tomato weight parameter was observed, far exceeding the control treatments and the commercial strain. With respect to the chlorophyll concentration parameter, significant differences were observed for the treatments compared to control in the seventh week. The treatment that had the best effect was the mixture of bacteria in the extended-release tablet presentation with 100% supplementary fertilization, exceeding 250 pg/mL of chlorophyll. The present invention describes a formulation and method that allows for increased plant growth capacity and better harvests.

## Definitions

The terms presented herein have the same meaning as is commonly used in the state of the art, unless otherwise stated. The terms used in this document are presented below:

The term "comprise" ("comprises," "comprising") and variations thereof, used in the description of this invention, both in the specification and in the list of claims, must be interpreted in an open and inclusive sense, that is, as "including, but not limited to."

When the term "obtained from" is used, it means that the sample is isolated or derived from a particular source. Likewise, the term "derived from" refers to the source from which the sample comes.

When the term "an embodiment" is presented, a particular feature or structure that has been described in connection with the embodiment of the invention is indicated, wherein at least one embodiment present therein is included, which do not necessarily correspond to the same embodiment or may be combined between them.

When the term "consisting of" is used, it indicates the list of elements within a phrase, which are mandatory and cannot be present in other elements. The term "essentially consists of" is used for any element listed after the phrase, this term being limited to other elements that do not interfere with the specific activity or action of the invention.

In other embodiments of the invention, the term "method" refers to a process for performing a task or obtaining a result of a given objective. The term "preventive method" refers to a process or set thereof that allows a task or objective to be performed in order to prevent a future event. The term "method of treatment" includes the process or set of these that are used to alleviate or cure a disease.

In other embodiments of the invention, when the term "wellness" or "plant well-being" is referred to, reference is made to the state of the plant in which its metabolism is operating optimally. On the other hand, when it refers to "production capacity" or "productive capacity" it is indicated as the maximum attainment of goods, in this case, it would be the maximum attainment of growing plants, the maximum capacity to obtain harvest, etc. In the case of "increase in production capacity," it refers to the significant increase in the number of plants, or an increase in harvest or increase in plant growth.

"Biological control" means a method for the control of pests, diseases and/or weeds intended to use living organisms for the control of growth of other populations of living organisms.

The term "Biocontroller" refers to products of non-synthetic origin that are used for pest control in crops.

When the document refers to the term "biostimulant," it is understood as a substance or mixture of substances with or without microorganisms that can be applied to crop plants, seeds, or roots. These substances or mixtures have the characteristic of improving aspects of the plant, such as obtaining better harvests, through stimulation of biological processes, improvements in nutrient availability, improvements in nutrient absorption, stress tolerance, etc.

When referring to the term "bioprotectant," this describes any product or device that is intended to protect the characteristics and functions of living things from external aggressions to which they may be subjected. An example of this in plants is when plants are subjected to abiotic stress.

When referring to "Organisms for Biological Control," this refers to microorganisms that have the ability to inhibit the growth of another microorganism and are used for pest or disease control.

When the term "Biofertilizer" is presented, this term refers to an agricultural input formulated from at least one growth-promoting bacteria that enhances crop performance when applied to it.

The term "PGPR" corresponds to the abbreviation "plant growth promoting rhizobacteria" and refers to bacteria present in the rhizosphere that colonize the root system of plants or their nearest environment and enhance the growth thereof. When referring to the term "Rhizobacteria," this corresponds to those bacteria present in the rhizosphere. When it is noted that a bacteria has PGPR activity, it means that said bacteria is able to colonize the root system of plants or their nearest environment and enhance the growth of the plant.

When the term "excipient" is presented, it refers to any substance that can be used without affecting the active ingredient. When referring to an "agriculturally acceptable excipient," this refers to any substance used to generate, produce or manufacture an agricultural product containing an active ingredient.

When referring to the term "tablet," it is indicated as a small item consisting of moldable material or ingredient, which can have various sizes, shapes and uses. In other embodiments of the invention, the term "Effervescent Tablet" refers to a tablet that within its composition generally contains acidic substances and carbonates or bicarbonates, which react rapidly in the presence of with the release of carbon dioxide. In other embodiments of the invention, the term "extended-release tablet" refers to a tablet having characteristics that allow the release of the active ingredient of a formulation in a slow manner, allowing the effect of the active ingredient to be extended between doses.

In other embodiments of the invention, the term "wettable powder" refers to those substances (active ingredients and/or excipients) present in a formulation that, when mixed with water prior to application, form a suspension.

In other embodiments of the invention, the term "resuspension" refers to the incorporation of material or substance which is suspended in liquid when it is found as a precipitate or as a dried material or substance.

In other embodiments of the invention, the term "emulsion" refers to a milky liquid containing small particles or droplets of another insoluble substance in the suspension.

Description of Figures of the Invention

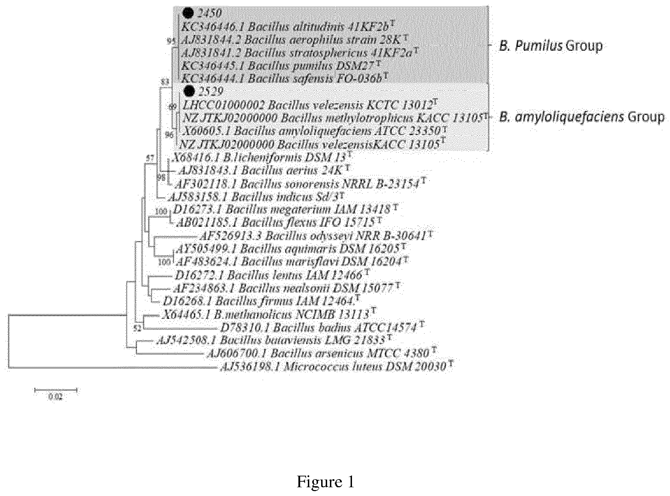

Figure 1. Phylogenetic tree based on 16S rDNA gene. The Neighbor-joining method and K-2 model parameters were used to determine the phylogenetic relationship between *Bacillus* sp. isolate and other *Bacillus* species. *Micrococcus luteus* DSM 20030T was used as an external group. Bootstrap values (expressed as percentages of 1000 replicates) greater than 50% are displayed on nodes. The bar indicates 2 substitutions per 100 nucleotides. The RGM 2450 and RGM 2529 isolates are highlighted with a black circle. On the left side of each type strain (T) is the accession number for the NCBI database.

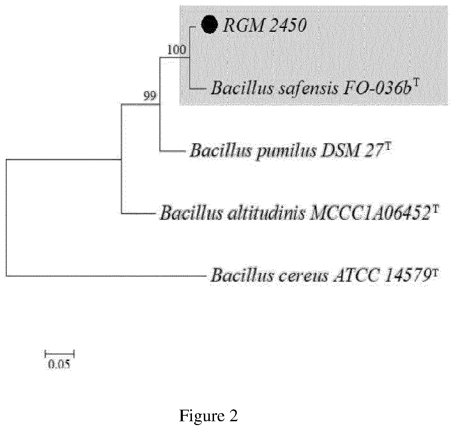

**Figure 2. Phylogenetic tree based on pyrE analysis of strain RGM 2450 sp. and related species types.** For pyrE genetic marker testing. The Maximum Likelihood method and the Tamura-Nei model were used to determine the phylogenetic relationship between RGM 2450 isolate and strains of the species *B. safensis, B. pumilus* and *B. altitudeinis*. The *B. cereus* ATCC 1479T strain was used as an external group. Bootstrap values (expressed as percentages of 1000 replicates) are displayed on nodes. The bar indicates 5 substitutions per 100 nucleotides. The RGM 2450 strain is highlighted with a black circle.

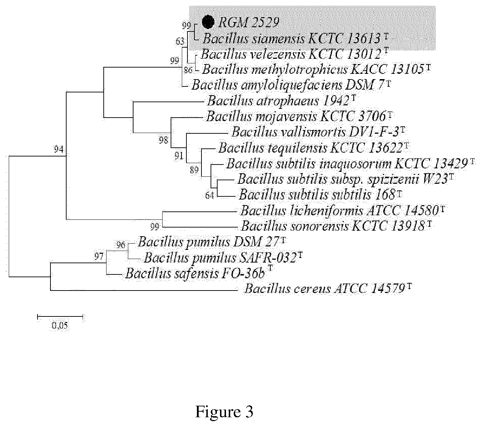

**Figure 3. Phylogenetic tree based on gyrB analysis of RGM 2529 strain and related species types.** The Maximum Likelihood method and Tamura-3-parameter model were used to determine the phylogenetic relationship between the RGM 2529 strain and the type strains. *B. cereus* ATCC 14579 was used as an external group. Bootstrap values (expressed as percentages of 1000 replicates) are displayed on nodes. The bar indicates 5 substitutions per 100 nucleotides.

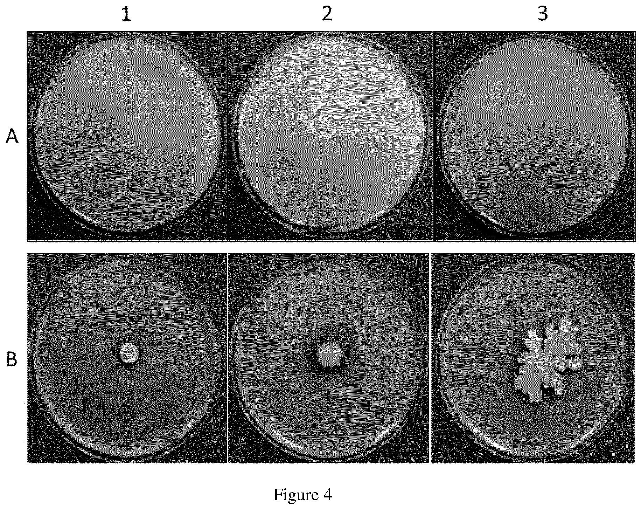

**Figure 4. Evaluation of Nitrogen (N2) binding and phosphorus (P) solubilization of RGM 2450, RGM 2529 and commercial strain QST 713.** The different bacterial strains were cultured in Ashby medium to test for N2 binding, whereas the assay in modified Pikovskaya medium was applied to evaluate P solubilization. The brightness of the photographs was adjusted by 60% from the original shot. The negative control is not shown in the figure; A) indicates nitrogen binding assays, B) indicates phosphorus solubilization assays, while 1) represents assays in the RGM 2450 strain, 2) assays in the RGM 2529 strain, and 3) assays with a commercial product.

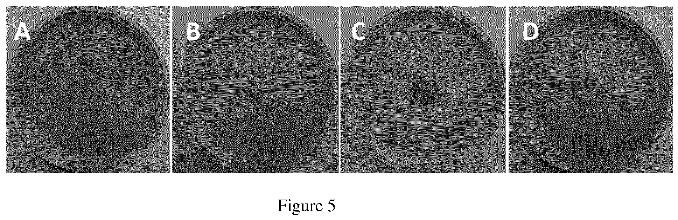

**Figure 5. Evaluation of Potassium (K) solubilization of RGM 2450, RGM 2529 strains compared to commercial strain QST 713.** All strains were cultured in modified Pikovskaya medium. A) Bacteria-free control plate; B) Plate cultured with RGM 2450 strain; C) Plate cultured with RGM 2529 strain; D) Plate cultured with QST 713 commercial product strain. The brightness of the photographs was adjusted by 60% from the original shot.

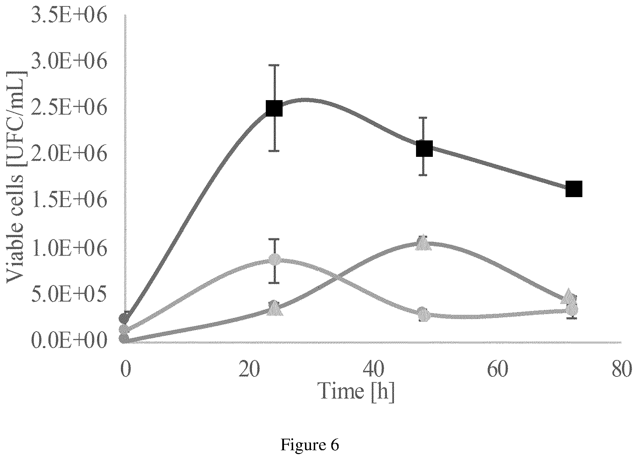

**Figure 6. Nitrogen binding for RGM 2450, RGM 2529 and QST 713 strains in modified Ashby medium (Nitrogen-free).** The strains were cultured in Ashby medium in the absence of N2 sources to assess their binding capacity over time. The lines represent the evolution in the number of viable cells for the RGM 2450 (■), RGM 2529 (▲) and QST 713 (▒) strains.

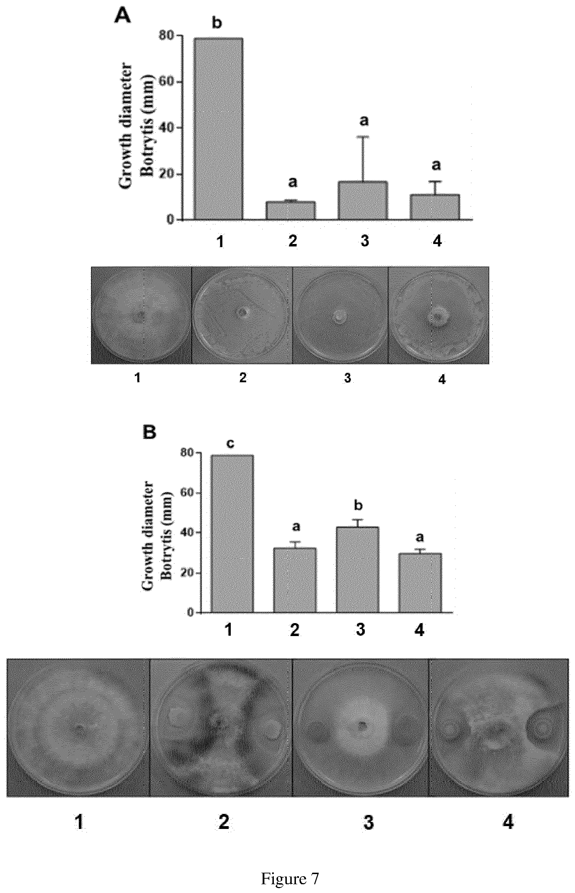

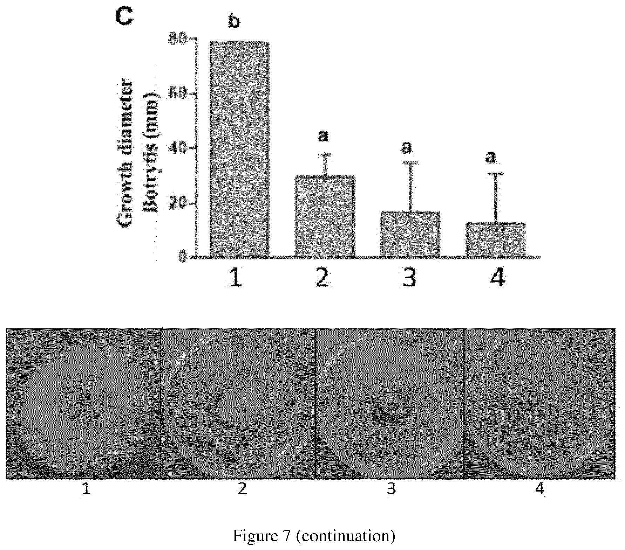

**Figure 7. Antagonist activity of RGM 2450, RGM 2529 and commercial strain QST 713 against phytopathogen *Botrytis* sp**. A) Antagonistic activity from direct pathogen contact with all three strains separately; B) Antagonistic activity from antifungal factor secretion; C) Antagonistic activity from antifungal volatile compound secretion. The graphs show the growth diameter in millimeters (mm) of *Botrytis* sp, where 1) corresponds to the control (untreated *Bottrytis*), 2) corresponds to a commercial product vs. *Botrytis,* 3) RGM 2450 vs. *Botrytis,* 4) RGM 2529 vs. *Botrytis.* Average values are expressed along with their respective standard deviation. Letters indicate significant differences (p-value < 0.05, ANOVA, LSD).

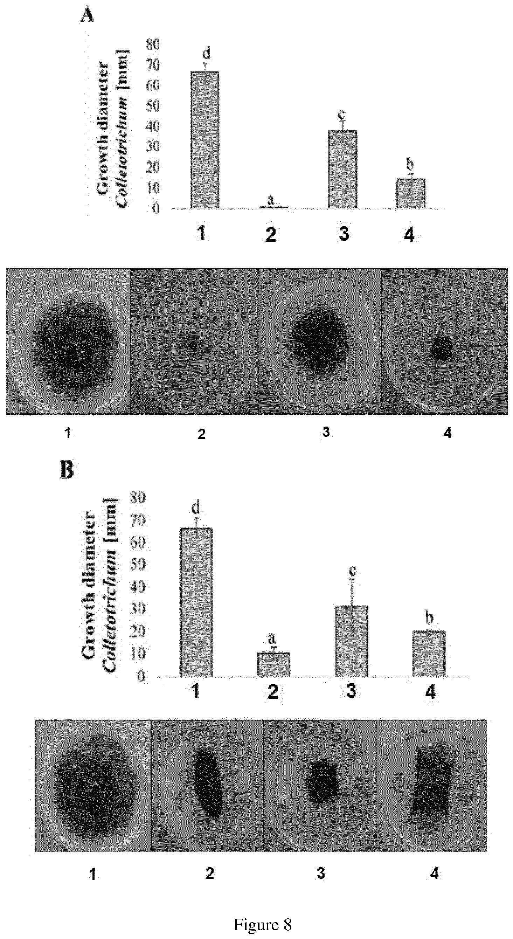

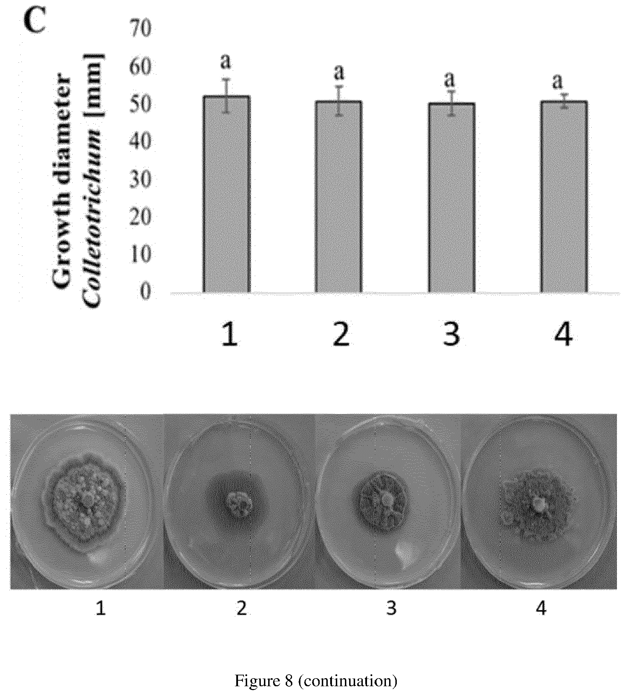

**Figure 8. Antagonist activity of RGM 2450, RGM 2529 and commercial strain QST 713 against the phytopathogen *Colletotrichum* sp.** A) Antagonistic activity from direct pathogen contact with all three strains separately; B) Antagonistic activity from antifungal factor secretion; C) Antagonistic activity from antifungal volatile compound secretion. The graphs show the growth diameter in millimeters (mm) of *Colletotrichum* sp, where 1) corresponds to the control (untreated *Colletotrichum*), 2) corresponds to a commercial product vs. *Colletotrichum,* 3) RGM 2450 vs. *Colletotrichum,* 4) RGM 2529 vs. *Colletotrichum.* Average values are expressed along with their respective standard deviation. Letters indicate significant differences (p-value < 0.05, ANOVA, LSD).

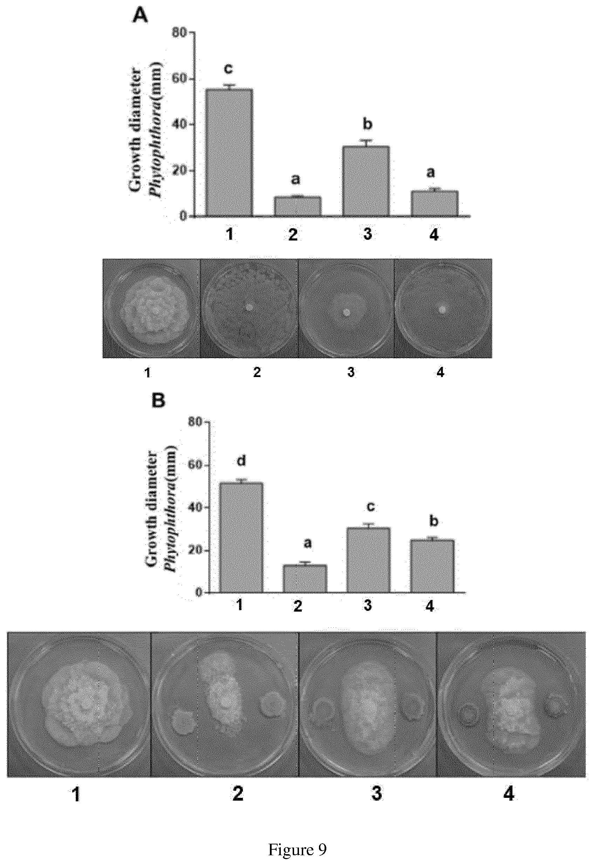

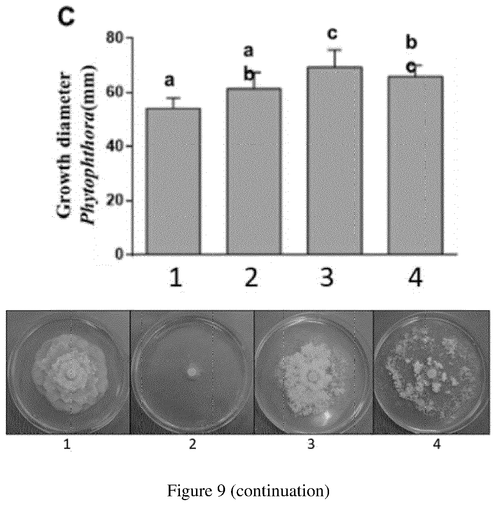

**Figure 9. Antagonist activity of RGM 2450, RGM 2529 and commercial strain QST 713 against *Phytophthora* sp.** A) Antagonistic activity from direct pathogen contact with all three strains separately; B) Antagonistic activity from antifungal factor secretion; C) Antagonistic activity from antifungal volatile compound secretion. The graphs show the growth diameter in millimeters (mm) of *Phytophthora* sp., where 1) corresponds to the control (untreated *Phytophthora*), 2) corresponds to a commercial product vs. *Phytophthora,* 3) RGM 2450 vs. *Phytophthora,* 4) RGM 2529 vs. *Phytophthora.* Average values are expressed along with their respective standard deviation. Letters indicate significant differences (p-value < 0.05, ANOVA, LSD).

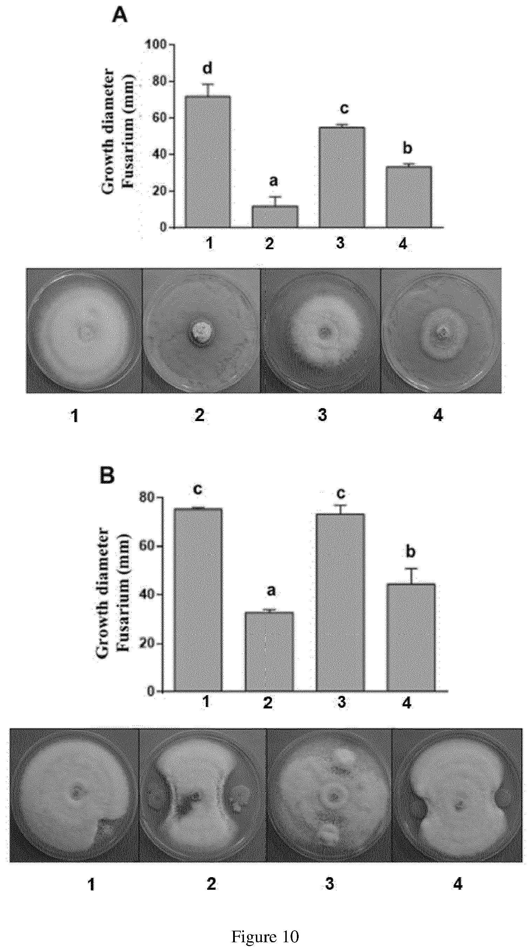

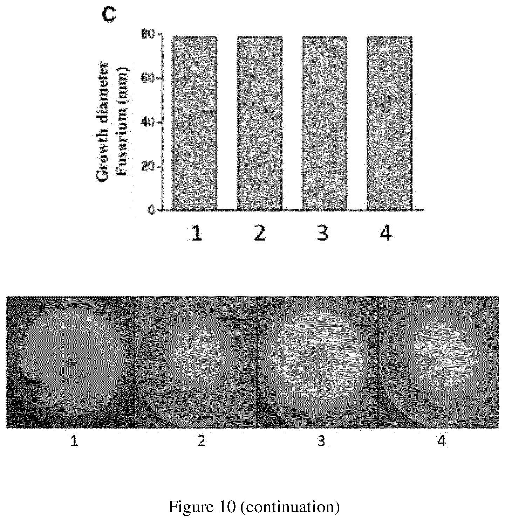

**Figure 10. Antagonist activity of RGM 2450, RGM 2529 and commercial strain QST 713 against the phytopathogen *Fusarium* sp.**A) Antagonistic activity from direct pathogen contact with all three strains separately; B) Antagonistic activity from antifungal factor secretion; C) Antagonistic activity from antifungal volatile compound secretion. The graphs show the growth diameter in millimeters (mm) of *Fusarium* sp., where 1) corresponds to the control (untreated *Fusarium*), 2) corresponds to a commercial product vs. *Fusarium,* 3) RGM 2450 vs. *Fusarium,* 4) RGM 2529 vs. *Fusarium.* Average values are expressed along with their respective standard deviation. Letters indicate significant differences (p-value < 0.05, ANOVA, LSD).

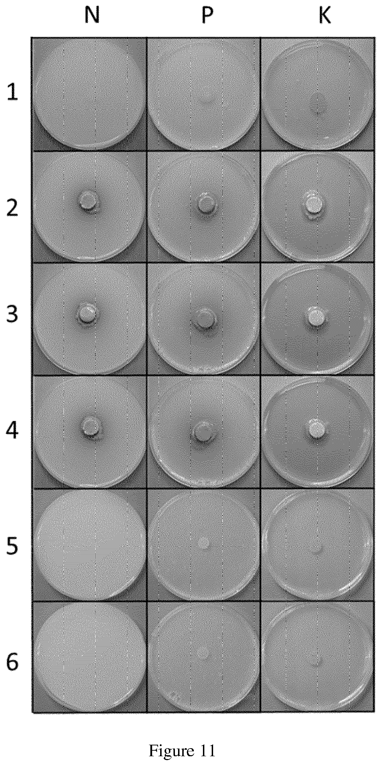

**Figure 11. Evaluation of N2 binding activities and P and K solubilization of the RGM 2450 strain in the different formulation formats.** Figure presents the different assays in which nitrogen (N) binding and phosphorus (P) and potassium (K) solubilization of the RGM 2450 strain were evaluated. Different presentation formats for the formulation are evaluated, where 1) corresponds to the control, 2)-3)-4) correspond to the extended-release tablet formulation without gelatin, 0.5X gelatin and 1X gelatin, respectively, 5) wettable powder, and 6) effervescent tablet.

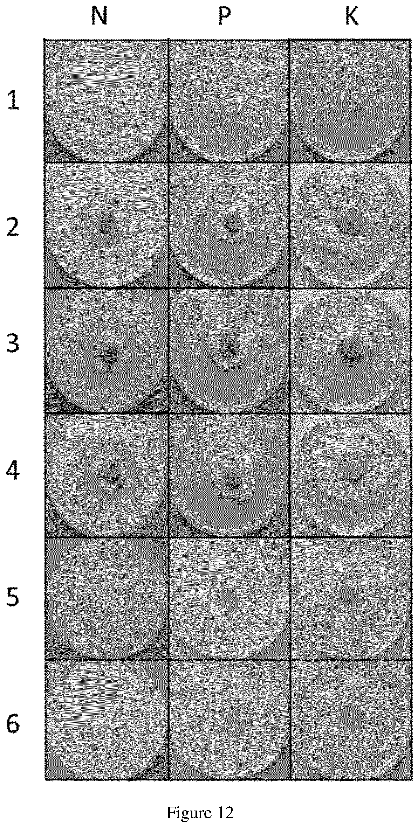

**Figure 12. Evaluation of N2 binding activities and P and K solubilization of the RGM 2529 strain** in the different formulation formats. Figure presents the different assays in which nitrogen (N) binding and phosphorus (P) and potassium (K) solubilization of the RGM 2529 strain were evaluated. Different presentation formats for the formulation are evaluated, where 1) corresponds to the control, 2)-3)-4) correspond to the extended-release tablet formulation without gelatin, 0.5X gelatin and 1X gelatin, respectively, 5) wettable powder, and 6) effervescent tablet.

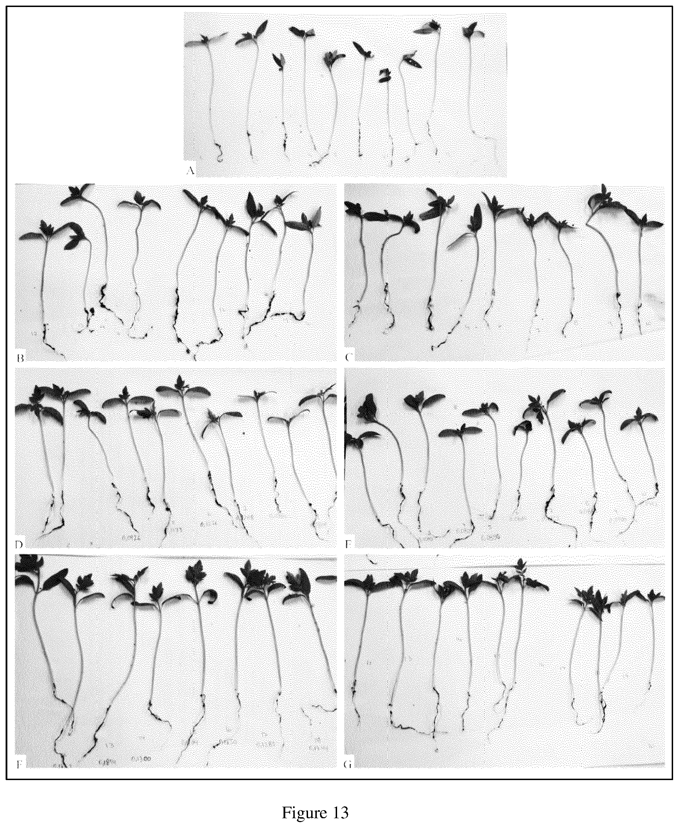

**Figure 13. Effect of RGM 2450 and/or RGM 2529 strains on tomato seedling growth.** The evaluation was performed 21 days after inoculation of the seeds in the described treatments. A) Control; B) Resuspension of RGM 2450 strain in isotonic solution; C) Emulsion of RGM 2450 strain; D) Resuspension of RGM 2529 strain in isotonic suspension; E) Emulsion of RGM 2529 strain; F) Resuspension of RGM 2450 and 2529 strain mixture in isotonic solution; G) Emulsion of RGM 2450 and 2529 strain mixtures.

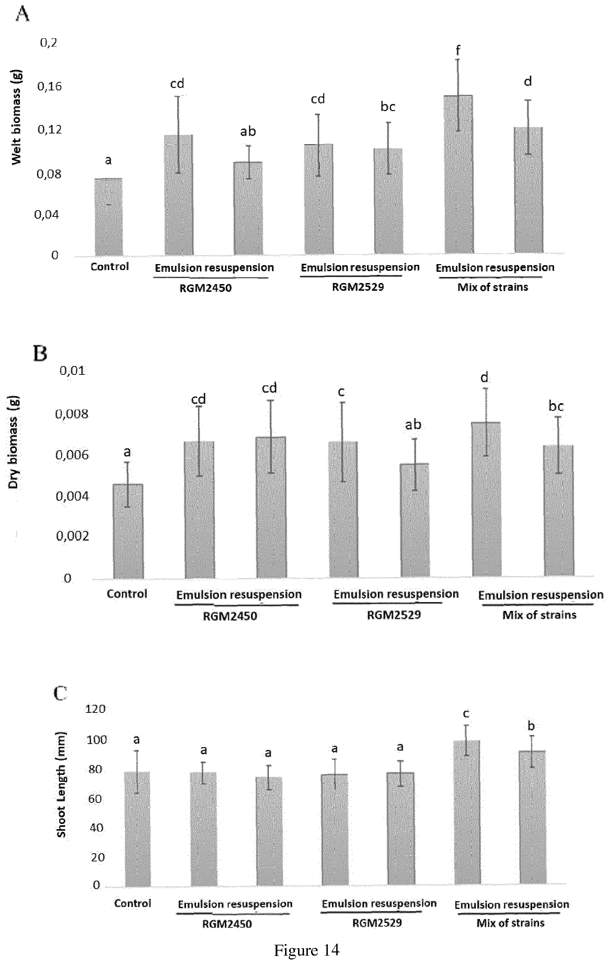

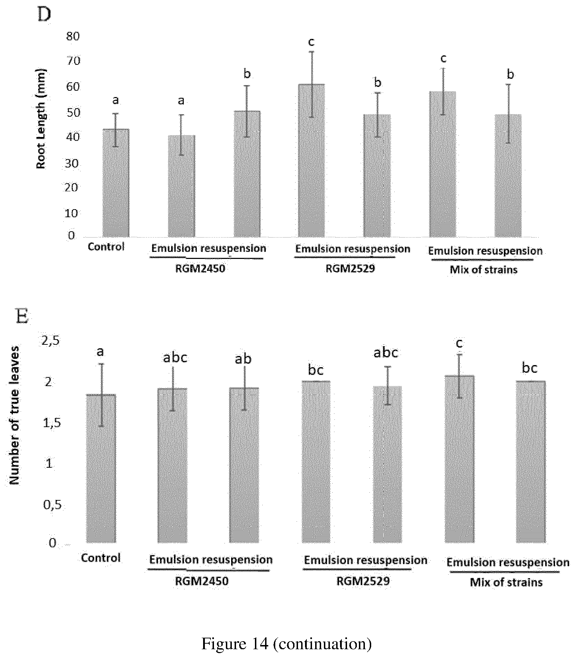

**Figure 14. Evaluation of the growth of tomato seedlings from seeds inoculated with bacteria.** The evaluation was performed 21 days after inoculation of the seeds in the described treatments. A) Wet biomass assessment; B) Dry biomass assessment; C) Outbreak length assessment; D) Root length assessment; E) True leaf count assessment. Different letters indicate that there are significant differences between treatments (p-value < 0.05, ANOVA, LSD).

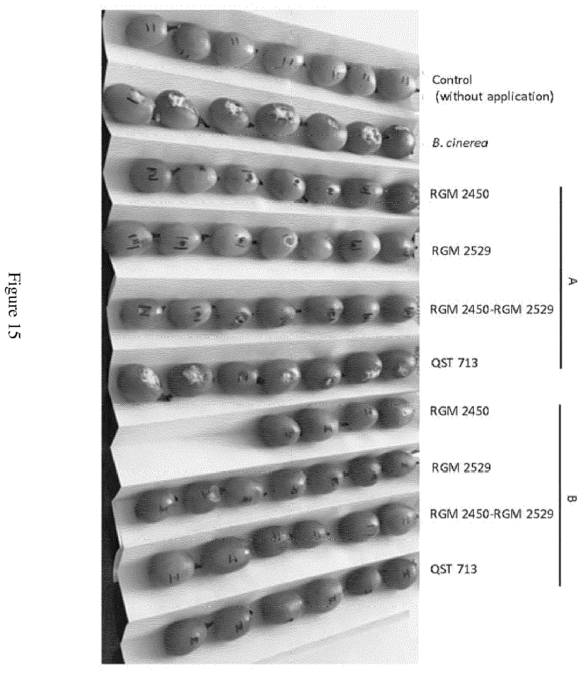

**Figure 15. Antagonist activity of PGPR strains against *B. cinerea* in grape berry** assays. The photo is a representative snapshot of the activity of the RGM 2450 and RGM 2529 strains, the mixture of both, and the commercial strain QST 713. A) indicates berries inoculated with *B. cinerea* and B) indicates berries not inoculated with *B. cinerea.* The photograph was taken after incubating the experimental units for 72 hours at 25°C and high humidity.

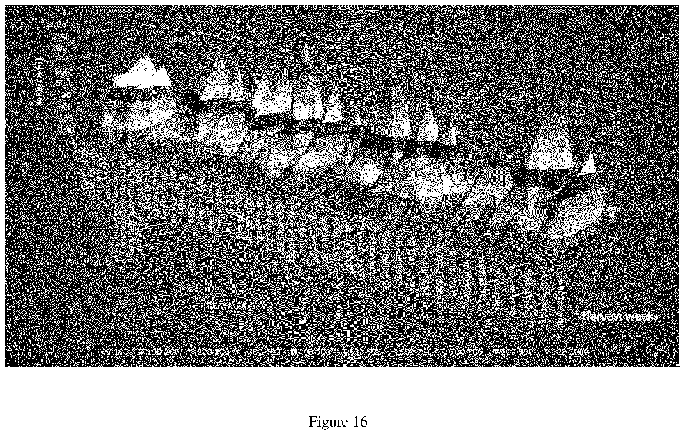

**Figure 16. Cumulative weight response surface of tomato fruits.** On the Y axis is the cumulative weight (g) in X treatments and Z weeks. The colors represent the different surfaces covered according to the g achieved in each treatment.

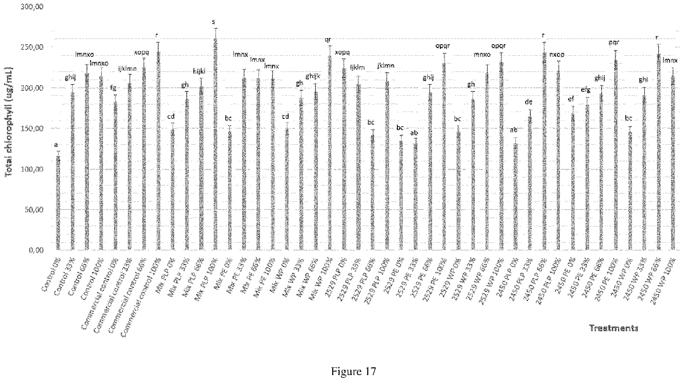

**Figure 17. Quantification of total chlorophyll with respect to each treatment.** The bars represent the average values. Lines represent standard deviations and the letters the LSD test ($\alpha$=0.05).

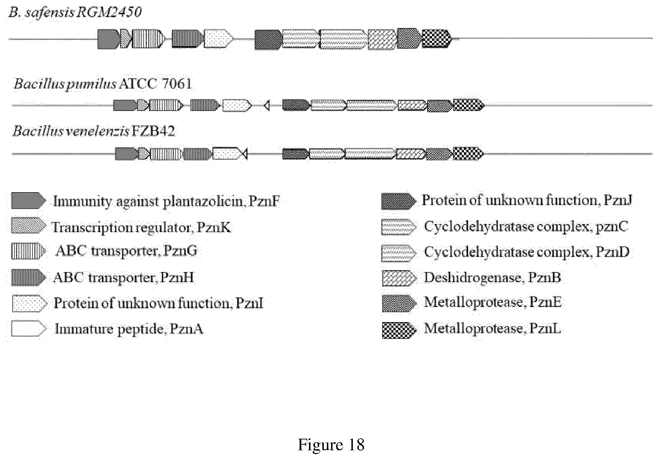

**Figure 18. Grouping involved in the synthesis and transport of the microfine plantazolicin.** The gene cluster of the RGM 2450 strain is compared to the gene cluster of the *B. pumilus* ATCC 7061 and *B. venelenzis FZB42* strains involved in the synthesis of plantazolicin.

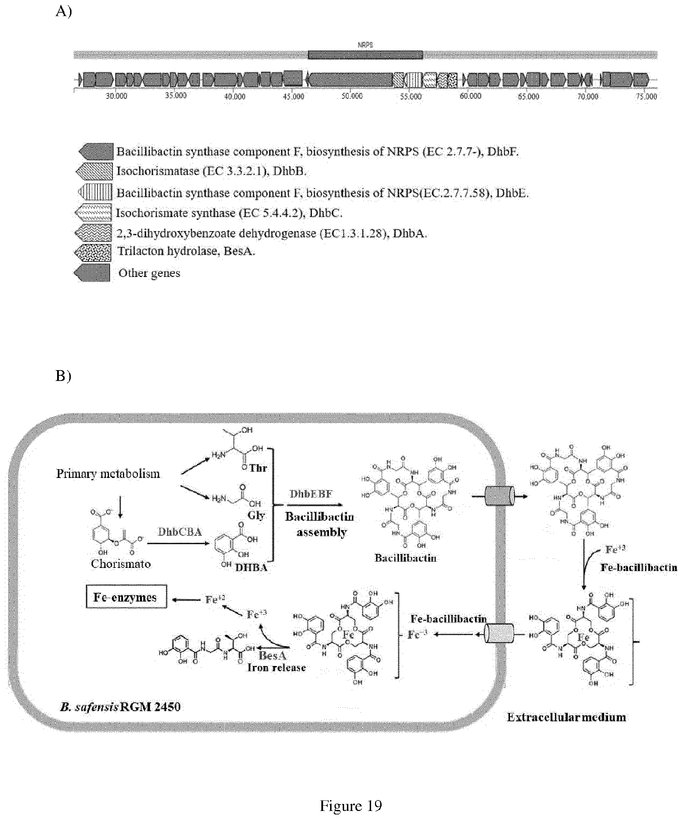

**Figure 19. Prediction of gene cluster encoding non-ribosomal peptide synthetase and other enzymes that would be involved in the synthesis of bacillibactin.** A) Gene cluster encoding enzymes involved in the synthesis of bacillibactin and adjacent genes involved in transport and regulation functions. B). Schematic plotting the role of enzymes in the synthesis of bacillibactin and the role of this compound. Bacillibactin is synthesized by an NRPS assembly system (DhbACEBF) and secreted into the extracellular space. Bacillibactin chelates iron with very high affinity, and the resulting ferric-bacillibactin complex is imported back into the cytosol via the FeuABC-YusV system and hydrolyzed by BesA esterase to release iron, which produces three bacillibactin monomers (2,3 - dihydroxybenzoate-Gly-Thr). The released iron serves as an enzyme cofactor.

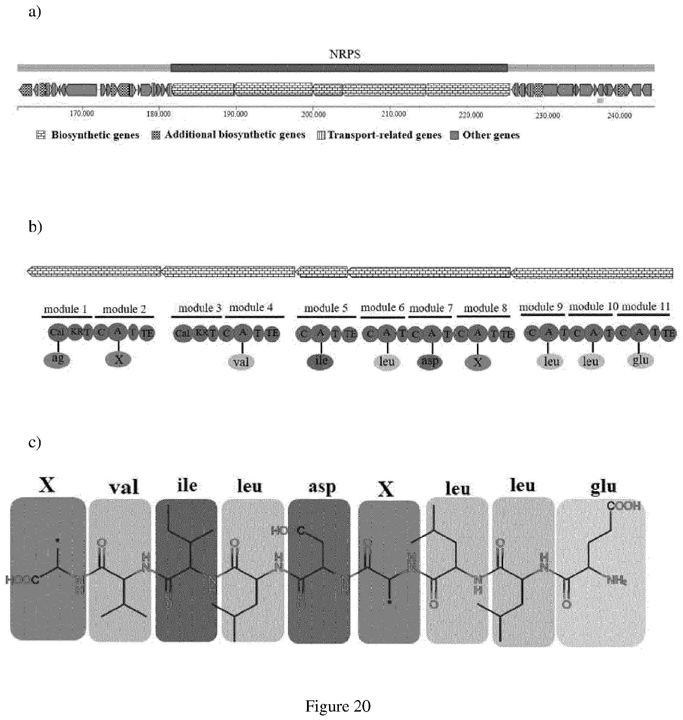

**Figure 20. Prediction of gene cluster encoding NRPS that would be involved in the synthesis of cyclolipopeptide.** A) genes encoding NRPS and its gene neighborhood. B) prediction of non-ribosomal peptide synthetase encoded by biosynthetic genes along with amino acids that are recognized by their adenylation domains. Cal, Co-enzyme A ligase domain, Ag, fatty acid; X, **identified amino acid;** val, valine; le, [sic] isoleucine; leu, leucine; asp, aspartate; glu, glutamic acid. Partial prediction of molecule synthesized by NRPS.

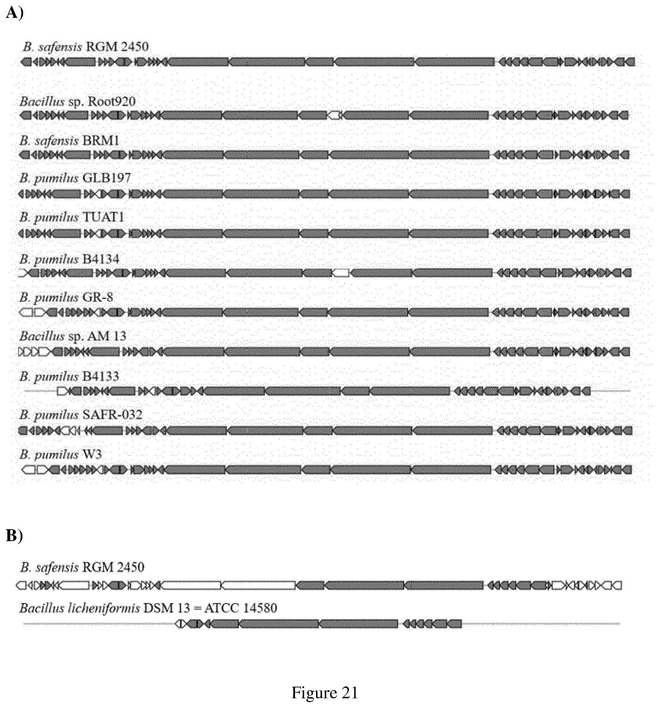

**Figure 21. Gene cluster arrangement that would be involved in the synthesis of cyclolipopeptide in strains of the genus *Bacillus sp.*** A) Gene cluster arrangement in strains phylogenetically close to the RGM 2450 strain. B) Comparison of the RGM 2450 strain gene cluster with that of the B. *licheniformis* DSM 13 strain involved in the synthesis of the cyclolipopeptide of lichenysin.

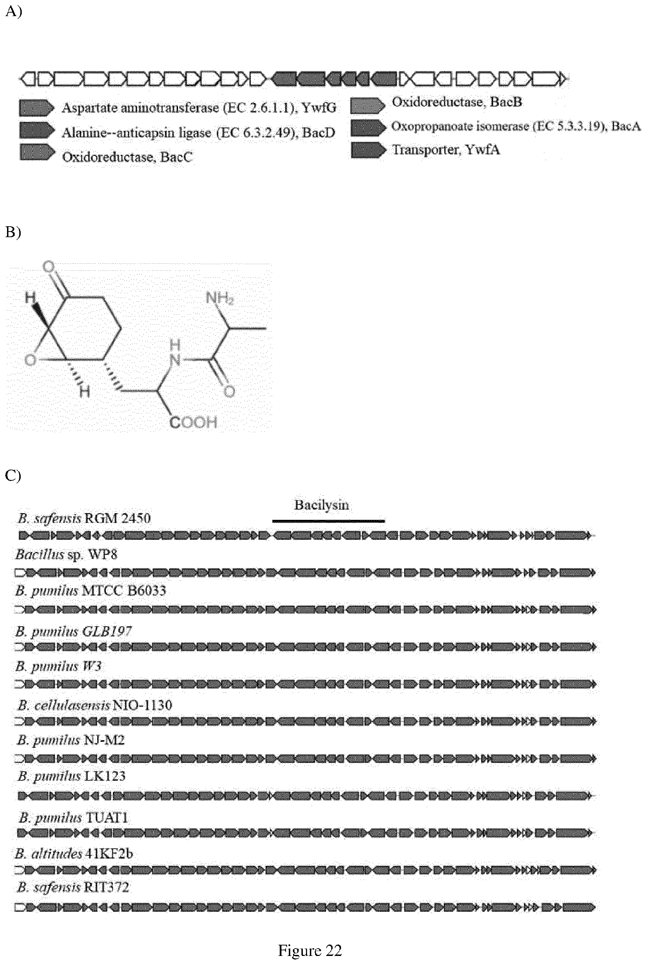

**Figure 22. Prediction of gene cluster encoding NRPS that would be involved in bacilysin synthesis.** A) Genes involved in the synthesis and transport of the bacilysin peptide are illustrated. B) Bacilysin structure. C) Gene cluster arrangement in other strains phylogenetically close to the RGM 2450 strain.

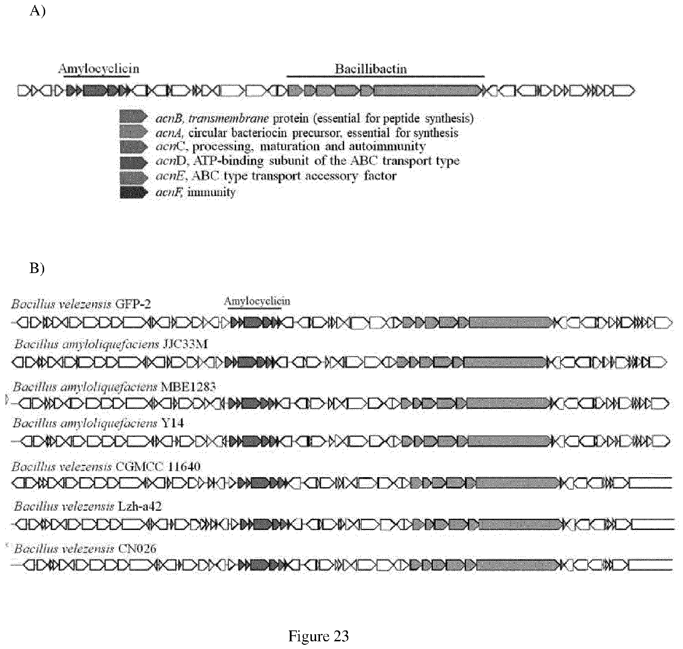

**Figure 23. AcnABCD gene cluster encoding amylocyclicin.** A) Gene cluster encoding amylocyclicin in the RGM 2529 strain. B) Presence of gene cluster in strains of PGPR species.

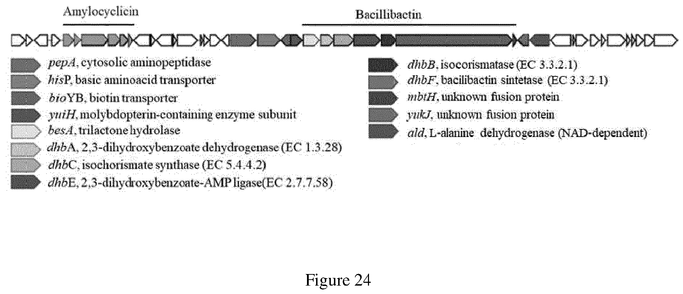

**Figure 24. DhbACEBF gene cluster of RGM 2529 strain involved in bacillibactin synthesis.** The 13034 bp DhbACEBF gene cluster involved in the synthesis and assembly of the bacillibactin siderophore was detected in the vicinity of the amylocyclicin gene neighborhood.

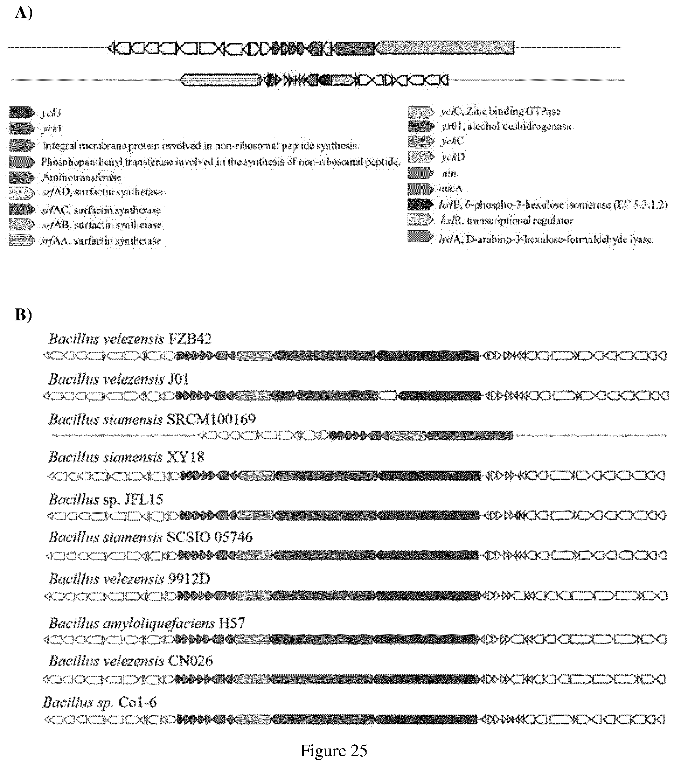

**Figure 25. Gene cluster that would be involved in the synthesis of surfactin.** A) Gene cluster arrangement of surfactin in the RMG 2529 strain. B) Gene cluster of surfactin in strains phylogenetically close to the RGM 2529 strain.

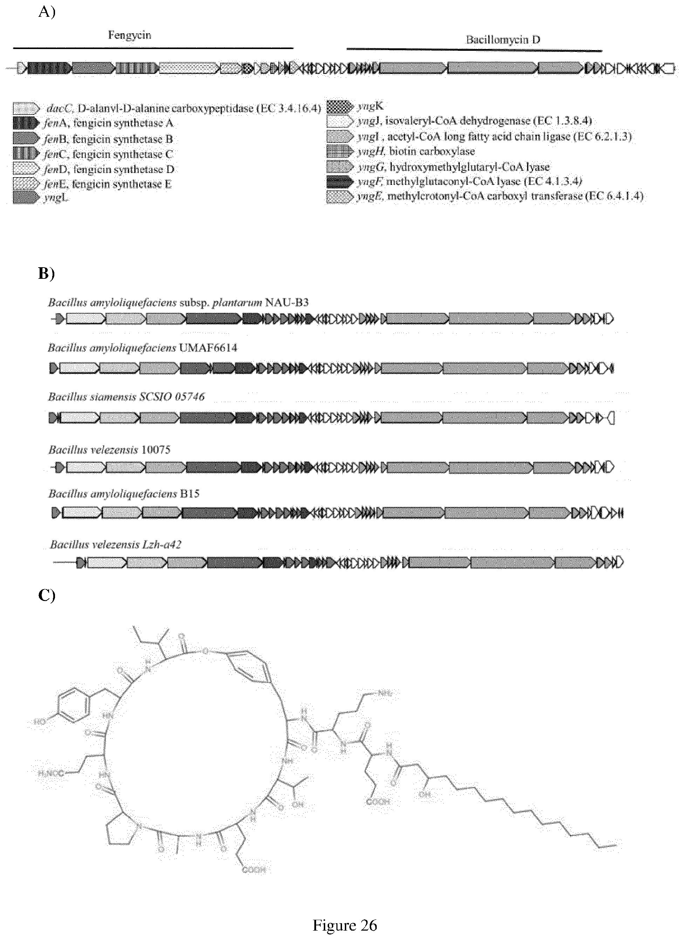

**Figure 26. Gene cluster that would be involved in the synthesis of fengycin.** A) Gene cluster arrangement of fengycin in the RMG 2529 strain. B) Gene cluster of fengycin in strains phylogenetically close to the RGM 2529 strain. C) Fengycin structure.

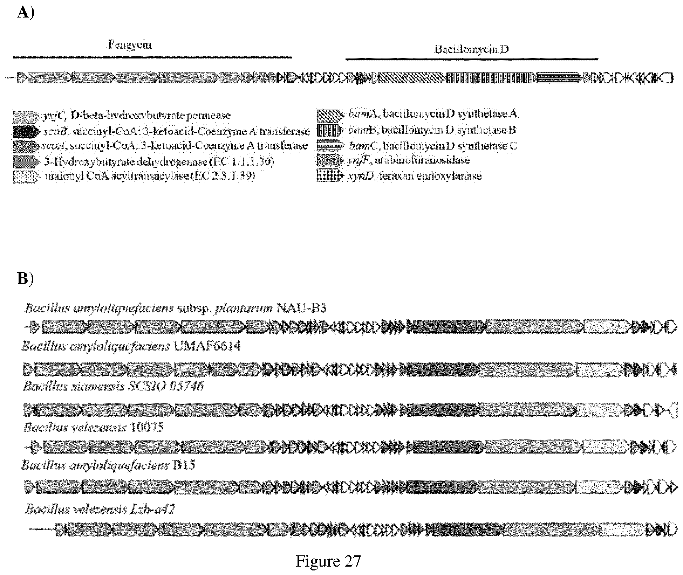

**Figure 27. Gene cluster that would be involved in the synthesis of bacillomycin D.** A) Gene cluster arrangement of bacillomycin D in the RMG 2529 strain. B) Gene cluster of bacillomycin D in strains phylogenetically close to the RGM 2529 strain.

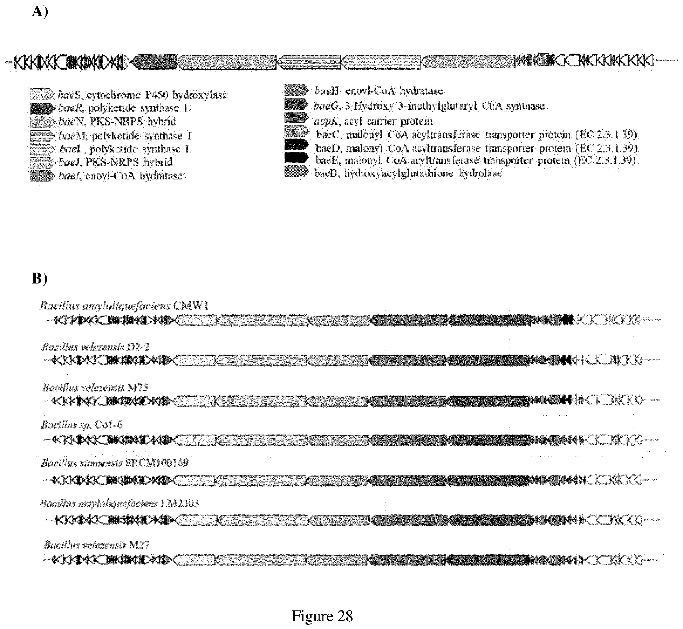

**Figure 28. Gene cluster that would be involved in the synthesis of bacillaene.** A) Gene cluster arrangement of bacilaene in the RMG 2529 strain. B) Gene cluster of bacilaene in strains phylogenetically close to the RGM 2529 strain.

## Application Examples

### Example 1. Characterization of bacterial strains RGM 2450 and RGM 2529.

The characterization of the bacterial strains RGM 2450 and RGM 2529, active ingredients of the formulation described herein, is presented in this application example. Molecular characterization and its characterization from the point of view of its nitrogen binding capacity and solubilization of phosphorus and potassium, and phytohormone production capacity (IAA) are described. Its ability to control pathogens is also evaluated.

1.1 Molecular characterization

DNA extraction and amplification of 16s, *pyrE and rpoB genes.*

DNA extraction was performed for RGM 2450 and RGM 2529 strains. Genetic markers were amplified: rRNA 16S and *pyrE* for the RGM 2450 strain, and rRNA 16S and *rpoB* for the RGM 2529 strain. The amplification reaction of each genetic marker consisted of 25 to 50 ng DNA from the strain, 1X DreamTaq Green PCR Master Mix, 400 $\mu$Mol from each splitter (Table 1), and nuclease-free Millipure water in a final reaction volume of 50 $\mu$L. The amplification thermal profile consisted of an initial denaturation at 95°C for 2 min, followed by 35 amplification cycles (denaturation at 98°C for 30 s, alignment at 55°C for 30 s, and an extension at 72°C for 45 s) and a final extension at 72°C for 5 min. PCR products were visualized by 1% agarose gel electrophoresis and GelRed 1X staining. The band corresponding to the expected size was cut and purified with the Zymo Clean Kit (Qiagen, Hilden, Germany) under the manufacturer's instructions. The purified fragment of this reaction was sent to sequence to the Macrogen sequencing center. Each marker was sequenced as sense and antisense strands, using the primers in Table 1. Sense and antisense sequences obtained from sequencing strain genetic markers were assembled using the Vector NTI program.

**Table 1. Sequences of primers used to amplify genes involved in molecular characterization of RGM 2450 and RGM 2529 strains.**

| Primer | Composition of the Primer | Blank Gene | References |
|---|---|---|---|
| 27F | AGAGTTTGATCMTGGCTCAG | rRNA 16S | Weisburg *et al.,* 1990 |
| 1492R | TACGGYTACCTTGTTACGACTT | | |
| *rpoBF* | GTTGGCTTCATGACTTGGGA | *rpoB* | Fan *et al.,* 2017 |
| *rpoBR* | ACGTTCCATACCTAAACTTTG | | |
| *pyrEF* | AGACCGTTCTTCCATCCA | *pyrE* | |
| *pyrER* | CACCTATTACAAATCAAAGC | | Liu *et al.,* 2013 |

Analysis of the identity and phylogenesis of the species.

The sequence of the RGM 2450 and RGM 2529 strain markers was compared to the species-type sequences of the bacterial groups to which they belong (Table 2). First, the identity of the RGM 2450 and 2529 strains was verified using the 16S rRNA genetic marker to determine the bacterial genus and group to which they belong, and subsequently using the specific markers *pyrE* and *rpoB* to corroborate the particular species of RGM 2450 and RGM 2529, respectively. Analysis of the 16S rRNA gene indicated that the RGM 2450 strain belongs to the group *B. pmilus,* while RGM 2529 belongs to the group *B. amyloliquefaciens.*

**Table 2. Nucleotide identity between strains RGM 2450 and RGM 2529 with bacteria of species *B. pumilus* and *B. amyloliquefaciens.*** In parentheses next to each species is their accession ID in the NCBI database.

| Strain Name and Accession ID | Locus | Identity (%) | Gaps (%) |
|---|---|---|---|
| RGM 2450 / *B. safensis* FO-036bT(AF234854.2) | *16S* | 99 | 0 |
| RGM 2450 / *B. pumilus* DSM 27$^T$ (KC346445.1) | | 99 | 0 |
| RGM 2450 / *B. safensis* FO-036b T(KC346688) | *pyrE* | 96 | 0 |
| RGM 2450 / *B. pumilus* DSM 27$^T$ (KC346746) | | 91 | 0 |
| RGM 2529 / *B. siamensis* KCTC 13613$^T$ (Y79DRAFT_AJVF01000001_1.1) | | 100 | 0 |
| RGM 2529 / *B. velezensis* KCTC 13012$^T$ (Ga0100777_104) | *16S* | 99 | 0 |
| RGM 2529 / *B. methylotrophicus* KACC 13105$^T$ (Ga0077492_1001) | | 99 | 0 |
| RGM 2529 / *B. amyloquefaciens* DSM 7$^T$ (NC_014551) | | 99 | 0 |
| RGM 2529 / *B. siamensis* KCTC 13613$^T$ (Y79DRAFT_AJVF01000001_1.1) | | 99 | 0 |
| RGM 2529 / *B. velezensis* KCTC 13012$^T$ (Ga0100777_104) | *rpoB* | 98 | 0 |
| RGM 2529 / *B. methylotrophicus* KACC 13105$^T$ (Ga0077492_1001) | | 98 | 0 |
| RGM 2529 / *B. amyloquefaciens* DSM 7$^T$ (NC_014551) | | 98 | 0 |

Phylogenetic analyses were then performed based on sequenced markers using MEGA 6.0.6 software. (Figures 1, 2 and 3). To do this, the RGM 2450 and RGM 2529 gene marker sequences were aligned with the reference strain sequences using the MUSCLE option. Subsequently, the evolutionary history was determined using a statistical method *(Maximum Likehood/Neighbor-joining)* and a nucleotide substitution model (Kimura-2-parameters/Tamura-Nei/Tamura-3-parameters model), considering a Gamma distribution to model differences in the rate of variation between sites. Phylogenetic relationships were hypothesized using 1000 bootstrap replicas. The phylogenetic trees clearly plot the results set forth in Table 2, indicating that the RGM 2450 strain shares a taxonomic clade with the *B. safensis* FO-036b bacterial strain (100% bootstrap) (Fig. 2), while RGM 2529 is in the same clade as with *B. siamensis* KCTC 13613$^T$ (99% bootstrap) (Fig. 3).

1.2 Solubilization of Phosphorus and Potassium and Nitrogen Binding Capacity

In the case of the evaluation of phosphate solubilization, strains RGM 2450 and RGM 2528 were grown in 5 mL of LB liquid medium for 16 h (30°C and 200 rpm). 10 μL of the culture was added to Pikovskaya agar medium (0.3 g/L NaCl; 0.3 g/L MgSO$_4$-7H$_2$O; 0.03 g/L MnSO$_4$-4H$_2$O; 0.3 g/L KCl; 0.5 g/L (NH$_4$)$_2$SO$_4$; 0.03 g/L FeSO$_4$ 7H$_2$O; 2 g/L Ca$_3$(PO4)$_2$; 10 g/L glucose; 0.5 g/L yeast extract; 15 g/L agar. Adjust to pH 7.0 (Vázquez *et al.,* 2000). It was incubated for 7 days at 30°C. The appearance of a clear halo around the bacterial colony indicated the ability to solubilize phosphate. The assay was performed in triplicate.

For the evaluation of potassium solubilization, strains RGM 2450 and RGM 2528 were grown in 5 mL of LB liquid medium for 16 h (30°C and 200 rpm). 10 μL of the culture was added to modified Pikovskaya agar medium (0.3 g/L NaCl; 0.3 g/L MgSO$_4$-7H$_2$O; 0.03 g/L MnSO$_4$-4H$_2$O; 2 g/L KNO$_3$; 0.5 g/L (NH$_4$)$_2$SO$_4$; 0.03 g/L FeSO$_4$ 7H$_2$O; 0.5 g/L Ca$_3$(PO4)$_2$; 10 g/L glucose; 0.5 g/L yeast extract; 15 g/L agar. Adjust to pH 7.0 (Vázquez *et al.,* 2000). It was incubated for 14 days at 30°C. The appearance of a halo around the bacterial colony indicated the ability to solubilize potassium.

To determine nitrogen binding capacity, strains RGM 2450 and RGM 2528 were cultured in 5 mL of LB liquid medium for 16 h (30°C and 200 rpm). It was centrifuged for 5 min at 3500 g and washed 3 times with sterile PBS buffer (8 g/L NaCl, 0.2 g/L KCl, 1.44 g/L Na$_2$HPO$_4$, 0.24 g/L KH$_2$PO$_4$, adjust to pH 7.4). Finally, 3 mL of PBS solution was resuspended and 10 μL of the suspension was added to Ashby medium (10 g/L glucose; 0.2 g/L KH$_2$PO$_4$; 0.2 g/L MgSO$_4$; 0.2 g/L NaCl; 0.2 g/L CaSO$_4$; 5 g/L CaCO$_3$) and incubated for 7 days at 30°C (Rao, 1999; Velázquez-Gurrola and Ramos-Alegria, 2015). Strain growth indicated the ability to bind atmospheric nitrogen. The assay was performed in triplicate.

As for P solubilization, the RGM 2450 strain exhibits a similar ability as the commercial product strain including the *Bacillus subtilis* QST 713 strain, while RGM 2529 exhibits a halo with greater range, demonstrating its superior ability to perform this task (Fig. 4). On the other hand, it is observed that both strains have the ability to solubilize K *in vitro* due to the color change in the medium (Fig. 5).

The RGM 2450 strain had the greatest effect on N$_2$ binding, with even better growth in modified Ashby medium.

For the N$_2$ binding assay (Fig. 6), it is observed that the RGM 2450 strain grew significantly better compared to the RGM 2529 and QST 713 strains after 24 hours of the experiment, demonstrating that its ability to bind this element is greater.

1.3 Phytohormone production capacity (IAA).

The ability of RGM 2450, RGM 2529 strains to produce indoleacetic acid phytohormone was determined. For this, strains RGM 2450, RGM 2529 and *B. subtilis* QST 713 were grown in 5 mL of LB medium at 30°C and 200 rpm. After 16 h of incubation, the turbidity of the cultures was measured at λ600 and an aliquot (λ600 turbidity = 1.0-1.2) of each strain was inoculated in LB culture media supplemented with tryptophan 1000 μg/mL (ALDRICH SIGMA). The relative IAA concentration in culture supernatants was determined by the Salkowski method (Patten and Glick, 2002; Yang *et al.,* 2007). Samples of 1.5 mL were collected and centrifuged at 3000 g for 10 min. An aliquot of 1 mL of supernatant was taken and vigorously mixed with 4 mL of Salkowski reagent (150 mL of concentrated H2O4, 250 mL of distilled H2O, 7.5 mL of 0.5 M FeCl3 × 6H2O) and incubated for 20 min at room temperature. The coloration formation indicated the presence of IAA, which was quantified by absorbance measurement at 535 nm. One calibration curve prepared with serial dilutions of IAA stock solution in LB medium was made at a concentration range of 0.7 and 12.5 pg/mL.

**Table 3. Concentration of IAA in bacterial cultures RGM 2450, RGM 2529 and QST 713.** The assay was performed on LB culture media containing tryptophan 1 mg/mL. Different letters represent significant differences between treatments *(p-value* < 0.05, ANOVA, Tukey).

| Strain | Absorbance λ = 535 | Relative IAA concentration [mg/mL] |
|---|---|---|
| RGM 2450 | 0.21 ± 0.01 | 0.0079 ± 0.0006[a] |
| RGM 2529 | 0.21 ± 0.01 | 0.008 ± 0.001[a] |
| QST 713 | 0.24 ± 0.01 | 0.0105 ± 0.0009[b] |

The results shown in Table 3 demonstrate that strains RGM 2450 and RGM 2529 are capable of producing IAA under the conditions delivered by the medium. There was no significant difference when comparing the concentrations produced by both strains. However, when compared to the commercial strain QST 713, both produced significantly less of this phytohormone: about 20% less after 48 hours of growth. It should be noted that high concentrations of IAA can result in inhibition of plant growth and deformation in plant tissue.

1.4 <u>Pathogen control capability.</u>

RGM 2450 and RGM 2529 strains were grown in LB culture medium for 16 h. A 100 pL aliquot of this culture was added to an LB agar plate and spread with the Drigalsky spatula over the entire surface of the agar. At the same time, a 6 mm diameter disc was removed from the edge of a culture of a resistant *Botrytis* strain, grown in PDA agar for 7 days. The disc was deposited in the center of a PDA agar plate, to evaluate the inhibition of phytopathogens by direct contact.

To determine inhibition by production of volatile compounds, the LB agar plate inoculated with a *Bacillus* strain was placed at the top of the plate inoculated with the phytopathogenic fungus. The bonded plates were sealed with 4 layers of Parafilm paper.

The same procedure was performed, using the phytopathogenic fungus *Colletotrichum.* It was left to incubate at room temperature for 7 days.

The percentage of inhibition of mycelium growth of pathogens tested by volatile and nonvolatile compounds was calculated according to the PGI formula:

$$PGI = ((C - T)/ C \times 100)$$

Where PGI = pathogen growth inhibition (%); C = pathogen diametrical growth (control); T = pathogen diametrical growth (treated).

According to the experimental design, ANDEVA analyses of variance were performed based on the F-test, with a p-value = 0.05 comparator, to determine the effect of exposure of nematodes to recombinant protein extracts. Finally, significant statistical differences were determined by LSD testing, p-value = 0.05.

**Table 4. Quantitative evaluation of the effect caused by strains RGM 2450 and RGM 2529 on *Botrytis sp.* growth** Values are expressed in percentages (%) of comparison in the pathogen growth halo measurement to the control group, along with their respective standard deviation.

| *Direct contact* | | | *Antibiosis* | | | *Volatile compounds* | | |
|---|---|---|---|---|---|---|---|---|
| | | QST | | | QST | | | QST |
| RGM 2450 | RGM 2529 | 713 | RGM 2450 | RGM 2529 | 713 | RGM 2450 | RGM 2529 | 713 |
| 79 ± 25 | 86 ± 7 | 90 ± 1 | 46 ± 5 | 62 ± 3 | 59 ± 4 | 79 ± 23 | 84 ± 23 | 62 ± 10 |

The results show that the antagonistic effect of the RGM 2450 strain against *Botrytis sp.* caused close to 79% inhibition upon direct contact with the pathogen, 46% by antibiosis, and 79% by volatile compound production; the RGM 2529 strain, in turn, exhibited 86%, 62%, and 84% inhibition upon exposure to the same fungus for direct contact, antibiosis, and volatile compound production assays, respectively (Table 4 and Figure 7).

**Table 5. Quantitative evaluation of the effect caused by strains RGM 2450 and RGM 2529 on *Colletotrichum sp.* growth** Values are expressed in percentages (%) of comparison in the pathogen growth halo measurement to the control group, along with their respective standard deviation.

| *Direct contact* | | | *Antibiosis* | | | *Volatile compounds* | | |
|---|---|---|---|---|---|---|---|---|
| RGM | | QST | RGM | | QST | RGM | | QST |
| | RGM | | | RGM | | | RGM | |
| 2450 | 2529 | 713 | 2450 | 2529 | 713 | 2450 | 2529 | 713 |
| 43 ± 9 | 78 ± 5 | 98 ± 0 | 53 ± 19 | 70 ± 2 | 84 ± 4 | N.D. | N.D. | N.D. |

On the other hand, for *Colletotrichum sp.,* the inhibition percentages observed for RGM 2450 were 43% and 53% for the direct contact and antibiosis assays, respectively; for RGM 2529, a decrease in the pathogen growth halo of 78% was observed by direct contact, and of 70% by antibiosis. No inhibitory effect was seen for volatile compounds in either strain (Table 5 and Figure 8).

**Table 6. Quantitative evaluation of the effect caused by strains RGM 2450 and RGM 2529 on *Phytophthora sp.* growth.** Values are expressed in percentages (%) of comparison in the pathogen growth halo measurement to the control group, along with their respective standard deviation.

| Direct contact | | | Antibiosis | | | Volatile compounds | | |
|---|---|---|---|---|---|---|---|---|
| RGM 2450 | RGM 2529 | QST 713 | RGM 2450 | RGM 2529 | QST 713 | RGM 2450 | RGM 2529 | QST 713 |
| 45 ± 5 | 80 ± 2 | 85 ± 1 | 41 ± 4 | 52 ± 2 | 75 ± 3 | 0 | 0 | 0 |

For testing against the pathogen *Phytophthora sp.,* the RGM 2450 strain caused 45% inhibition by direct contact and a strong antibiosis effect of 41%. Meanwhile, the RGM 2529 strain showed an inhibition effect of 80% by direct contact and 52% when treated for antibiosis. No inhibition by volatile compound production was exhibited in any case (Table 6 and Figure 9).

**Table 7. Quantitative evaluation of the effect caused by strains RGM 2450 and RGM 2529 on *Fusarium sp.* growth.** Values are expressed in percentages (%) of comparison in the pathogen growth halo measurement to the control group, along with their respective standard deviation.

| Direct contact | | | Antibiosis | | | Volatile compounds | | |
|---|---|---|---|---|---|---|---|---|
| RGM 2450 | RGM 2529 | QST 713 | RGM 2450 | RGM 2529 | QST 713 | RGM 2450 | RGM 2529 | QST 713 |
| 24 ± 2 | 53 ± 2 | 83 ± 7 | 4±3 | 41 ± 8 | 56 ± 2 | 0 | 0 | 0 |

Finally, for *Fusarium sp.,* strains RGM 2450 and RGM 2529 caused growth inhibition of 24% and 4% by direct contact, and 53% and 41% by antibiotic assay, respectively. No inhibition by volatile compound production was exhibited for any of the strains (Table 7 and Figure 10).

**Example 2. Preparation of formulations comprising RGM 2450 and RGM 2529 strains with PGPR activity for different presentation and administration formats**

The formulation of the present invention may be presented and administered as a formulation of an extended-release tablet, as wettable powders, or as effervescent tablets.

2.1 Formulation of extended-release tablets.

Three formulations were made to generate extended-release tablets, in order to determine dosage in secretion and preservation of viability. The first formulation consisted of 28.6% wheat flour, 28.6% cornstarch and 42.8% bacterial culture. The second formulation consisted of 30.4% wheat flour, 30.4% cornstarch, 38.2% bacterial culture, and 1% of 13% gelatin (0.5X gelatin).
The third formulation consisted of 32.6% wheat flour, 32.6% cornstarch, 32.8% bacterial culture, and 2% of 13% gelatin (1X gelatin). For formulation, the starch was dry sterilized at 180°C for 30 min and the gelatin was resuspended at a 1:8 ratio. The formulations were dosed in 0.62 g capsules with 1.4 cm diameter and 0.6 cm thickness. They were stored at 4°C for 16 h, then dehydrated at 40°C for 2 h. The tablets were deposited on 50 μL of water in the Ashby, Pikovskaya and modified Pikovskaya culture media, and incubated at 25°C for 14 days. Additionally, bacteria present in the formulations were counted by mashing and resuspending the formulation in 50 mL of sterile distilled water.

2.2 Formulation of wettable powders.

For the formulation of wettable powder, 17% potato starch was mixed with 14.6% silicon dioxide and 68.4% bacterial culture. 1g of the product was weighed and resuspended in 100 mL of sterile distilled water, 20 μL of the suspension was added in the Ashby, Pikovskaya and modified Pikovskaya culture media, and incubated at 30°C for 7 days. Additionally, bacteria present in the formulations were counted.

2.3 Formulation of effervescent tablets.

For the formulation of the effervescent tablets, 30% citric acid was mixed with 66.6% bicarbonate and 3.4% bacterial culture, and as the culture was added, homogenization was achieved by mixing the components. The resulting mixture was arranged in circular molds of 35 mm diameter and 0.5 mm depth, generating six 4.7 g tablets. They were dissolved in 100 mL of sterile distilled water and 20 pL of this solution were deposited in Ashby, Pikovskaya and modified Pikovskaya culture media, and incubated at 30°C for 7 days. Additionally, bacteria present in the formulations were counted.

**Table 8. Viable cell counts present in the different formulations.** The data correspond to the values in colony forming units (CFU) per gram of average formulation, together with their respective standard deviation, when performing the assay in triplicate.

| Viable cells (CFU/g formulation) | RGM 2450 strain | RGM 2529 strain |
| --- | --- | --- |
| Gelatin-free tablet | $1.0*10^7 \pm 2*10^6$ | $6*10^6 \pm 2*10^6$ |
| Tablet with 0.5X gelatin | $2.6*10^7 \pm 6*10^6$ | $7*10^6 \pm 3*10^6$ |
| Tablet with 1X gelatin | $8*10^6 \pm 1*10^6$ | $1.2*10^7 \pm 1*10^6$ |
| Wettable powder | $4*10^8 \pm 1*10^8$ | $4*10^7 \pm 3*10^7$ |
| Effervescent tablet | $5.3*10^6 \pm 6*10^5$ | $3.4*10^6 \pm 6*10^6$ |

According to Table 8, the number of viable cells for the five different formulations was between $3.4*10^6$ - $4*10^8$ CFU/g and was equal to or greater than the number of viable cells present in the commercial product Serenade® liquid ($1.4*10^7$ CFU/g), which includes the QST 713 strain, previously used as a comparative control. The formulations were also tested to evaluate whether they possess the same characteristics as previously tested for nitrogen binding and solubilization of phosphorus and potassium. In the case of RGM 2450 bacteria (Fig. 11), it maintains its ability to fix nitrogen but is unable to solubilize P when tested in extended-release tablets, while K solubilization is not clearly observed. The effect on nitrogen binding was not as easily noticeable for the other formulations, so it cannot be confirmed that the strain maintains its ability when incorporating it into a wettable powder or effervescent tablet. On the other hand, strain RGM 2529 (Fig. 12) was able to grow in the case of extended-release pills, mainly due to the formulation's own effect on the medium rather than a particular biological activity of the bacteria. There was no effect on the solubilization of P or K. In the wettable powders and the effervescent tablet, the conditions exhibited were the same as that of the control, so it is confirmed that the solubilization capacity of the strain is at least maintained in these two formulations.

**Example 3. Use of formulations comprising RGM 2450 and RGM 2529 strains as plant biostimulants/bioprotectants**.

3.1 Study of biostimulant effect *In vitro.*

Different treatments were applied to sterilized tomato seeds in the following bacterial resuspensions and emulsions: (i) resuspension of RGM 2450 strain, (ii) resuspension of RGM 2529 strain, (iii) mixture of both strains at a 1:1 ratio, (iv) emulsion of RGM 2450 strains, (v) emulsion of RGM 2529 strain, (vi) mixture of the strain emulsion at a 1:1 ratio.
To prepare for resuspension of bacteria in a NaCl solution, a colony of strains RGM 2450 and RGM 2529, each separate in 5mL of LB medium, was inoculated to generate the pre-inoculums of the respective cultures. Pre-inoculums were incubated at 30°C at 200 rpm, to a turbidity of 1.0 ($\lambda$ = 600 nm). Flasks with LB medium were then inoculated with a 1% aliquot of pre-inoculum (i.e., 800 $\mu$L pre-inoculum in 80 mL culture). RGM 2450 and RGM 2529 strains were grown for 20 h in LB medium at 30°C. A 25 mL aliquot of each culture was centrifuged at 2500 g for 10 minutes. Subsequently, the supernatant was removed and the cells were resuspended in an isotonic solution of 0.9% NaCl. Centrifugation, discard of the supernatant and resuspension in 0.9% NaCl solution was performed 3 times to remove residues from the culture medium. Finally, the cells were resuspended in a 0.9% NaCl solution supplemented with 0.01% Tween 20. The viable cell concentration of the resuspension of strains RGM 2450 and RGM 2529 in the isotonic NaCl solution was 4.7 $\times 10^9$ ($\pm 8 \times 10^8$) and $1.8 \times 10^8$ ($\pm 5 \times 10^7$) CFU/mL, respectively. The cell concentration of the RGM 2529 strain is underestimated due to the formation of filamentous aggregates (Report 5), which prevent accurately determining the number of cells present, and the highest CFU/mL value that can be obtained per batch was used for these. The cell concentration used was based on the concentration of CFU/mL present in the commercial products registered in Chile as biostimulants: TRIBAC BIO ($1 \times 10^9$CFU/mL, company ANASAC) and TIFI ($2 \times 10^8$ CFU/g, company Inpacta).
The bacterial emulsion described was prepared in two phases. In the first phase, 5.33 mL of oil and 0.22 mL of Tween 20 were added to a tube. The tube was stirred for 1 minute in the vortex. In the second phase, 0.47 mL of glycerol, 3.75 mL of Silwet and 5 mL of the bacterial suspension from the previous treatment were added with a concentration 20 times higher and stirred for 1 minute in the vortex. The contents of both tubes were then mixed and stirred for 1 minute. An aliquot of the homogenized mixture was taken and resuspended in 9 mL of sterile distilled water to obtain the diluted bacterial emulsion.

To apply the treatments, between 20 and 25 seeds were used per treatment including control. Seeds treated with bacterial resuspensions were incubated with bacterial cells for 45 min under gentle orbital agitation (100 rpm). The seeds that were treated with the bacterial emulsion were soaked for 5 min in the diluted emulsion. Seeds with the control treatment were treated with 0.9% NaCl.

The seeds of each treatment were sown in germination trays containing a mixture of 1:1 (v:v) autoclaved peat: vermiculite and incubated at 25°C $\pm$ 2°C with a photoperiod of 16 h light and 8 h dark (Mena-Violante and Olalde-Portugal, 2007; Vaikuntapu et al., 2014; Cordero et al., 2018). At 21 days of incubation, the following parameters were evaluated: wet biomass, dry biomass, root length, shoot length, tomato shoot length, and number of true leaves (Mena-Violante and Olalde- Portugal, 2007; Vaikuntapu et al., 2014; Cordero et al., 2018). To determine the dried biomass, the tomato seedlings were incubated at 80°C for 48 h. After incubation they were brought to room temperature and their mass was determined. The results of the determination of the replicate growth parameters for each treatment are indicated in the supplementary tables present in the report appendix.

According to the experimental design, ANDEVA analyses of variance were performed based on the F-test, with a *p-value* = 0.05 comparator, to determine the effect of experimental treatments compared to the control. Finally, significant statistical differences were determined by LSD test, *p-value* = 0.05.

**Table 9. Impact of the use of RGM 2450 and/or RGM 2529 strains on the development and growth of tomato seeds.** Dry and wet biomass values are expressed in grams (g) while the length of shoots and roots are expressed in millimeters (mm). The standard deviation for each parameter is indicated in parentheses.

| | Biomass | | Length | | No. of true leaves |
|---|---|---|---|---|---|
| **Treatment** | **Wet** | **Dry** | **Shoot** | **Root** | |
| **Control** | 0.07 | 0.004 | 77 | 43 | |
| | ($\pm$ 0.02) | ($\pm$ 0.001) | ($\pm$14) | ($\pm$7) | 1.8 ($\pm$ 0.4) |
| **Resuspension RGM 2450** | 0.11 | 0.006 | 76 | 41 | |
| | ($\pm$ 0.03) | ($\pm$ 0.002) | ($\pm$7) | ($\pm$7) | 2 ($\pm$ 0.2) |
| **Emulsion RGM 2450 strain** | | | | | |
| | 0.08 | 0.007 | 73 | 50 | |
| | ($\pm$ 0.01) | ($\pm$ 0.002) | ($\pm$8) | ($\pm$10) | 1.9 ($\pm$ 0.3) |
| **Resuspension RGM 2529** | 0.10 | 0.007 | 74 | 61 | |
| | ($\pm$ 0.03) | ($\pm$ 0.002) | ($\pm$10) | ($\pm$13) | 2 |
| **Emulsion RGM 2529 strain** | 0.10 | 0.005 | 75 | 49 | |
| | ($\pm$ 0.02) | ($\pm$ 0.001) | ($\pm$8) | ($\pm$9) | 1.9 ($\pm$ 0.2) |
| **Resuspension strain mixture** | 0.14 | 0.007 | 97 | 58 | |
| | ($\pm$ 0.03) | ($\pm$ 0.001) | ($\pm$10) | ($\pm$9) | 2.0 ($\pm$ 0.3) |
| **Emulsion strain mixture** | 0.11 | 0.006 | 89 | 49 | |
| | ($\pm$ 0.03) | ($\pm$ 0.001) | ($\pm$11) | ($\pm$11) | 2 |

**Table 10. Percent comparison of the growth parameters of the tomato seeds.** Values are expressed in percentages (%) difference between plants treated with different formulations of RGM 2450 and/or RGM 2529 versus the control group (untreated).

| | Comparison between experimental treatment vs. control (%) | | | |
|---|---|---|---|---|
| | Biomass | Length | | No. of true leaves |
| **Treatment** | **Wet** | **Shoot** | **Root** | |
| Resuspension RGM 2450 | 55 | -1 | -5 | 7 |
| Emulsion RGM 2450 strain | 19 | -5 | 17 | 5 |
| Resuspension RGM 2529 | 41 | -3 | 42 | 9 |

(continued)

| | Comparison between experimental treatment vs. control (%) | | | |
|---|---|---|---|---|
| | Biomass | Length | | No. of true leaves |
| Treatment | Wet | Shoot | Root | |
| Emulsion RGM 2529 strain | 36 | -2 | 14 | 6 |
| Resuspension strain mixture | 103 | 26 | 36 | 13 |
| Emulsion strain mixture | 62 | 16 | 15 | 9 |

Seedlings from seeds treated with bacterial resuspensions and emulsions after 21 days of growth showed a significant increase in wet and dry biomass compared to control treatment (Tables 9 and 10; Figures 13 and 14). The wet biomass of the experimental treatments showed an increase between 19% and 103%, while the evaluation of dry biomass indicated an increase between 19% and 63%. Regarding the length of the shoot, only the strain mixtures showed a significant increase, between 16% and 26%; the other treatments showed no significant differences compared to the control.

Seedlings inoculated with the experimental treatments, except for seed incubation treatment with the RGM 2450 strain suspension, had a significant increase in root length, between 15% and 42%, compared to the control. In the evaluation of the number of true leaves, the strain mixture treatments along with the resuspension treatment of the RGM 2529 strain had significant differences from the control.

It should be noted that the seeds inoculated with a mixture of the RGM 2450 and RGM 2529 strains in the NaCl solution (0.9%) showed the greatest increase in wet and dry biomass, shoot length, root length, and true leaves, obtaining 103%, 63%, 26%, 36%, and 13%, respectively, compared to the control treatment.

3.2 Study of bioprotectant effect *in vitro*.

200 grape berries were washed with 0.1% detergent and after 15 minutes of washing, the water with detergent was discarded and the berries were disinfected with sodium hypochlorite ($NaClO_3$) at a final concentration of 0.5%. Berries were incubated with this disinfection solution for 3 minutes, then rinsed with sterile distilled water and deposited in an absorbent towel inside the laminar flow hood to remove excess water. After this, 4 berries per pile were used, where each pile corresponded to a repetition containing 4 experimental units (berries). Treatments consisted of inoculation of 2 $\mu$L cell resuspension of *B. safensis* RGM 2450 ($10^{10}$ CFU/mL), *B. siamensis* RGM 2529 ($10^6$ CFU/mL), a mixture of both strains, commercial fungicide Serenade® (Bayer), and water. After 24 hours of inoculation with each treatment, 2 $\mu$L spore resuspension of *B. cinerea* ($10^6$ CFU/mL) was added. Inoculation was performed through a hole made in the berry with the tip of a syringe.

In a second trial, the same treatments were applied, but the commercial product's capacity was evaluated without the excipients contained in its formulation. For this, 1 mL of product was centrifuged at 5000 g for 10 minutes and the supernatant was discarded to obtain the active bacterial compound (*B. subtillis* QST 713 strain). The pellet was resuspended with 1 mL of distilled water to obtain cell suspension. Subsequently, 2 $\mu$L of this solution was used to inoculate the corresponding experimental units.

After inoculation with *B. cinerea,* berries from both assays were incubated for 72 hours at 25°C and high humidity, then the damage caused by the phytopathogen in the fruits was qualitatively assessed (Figure 15 and Table 11).

**Table 11. Evaluation of the antagonistic activity of PGPR strains against *B. cinerea* in grape berries.** Diameters are expressed as the average lesion diameter, along with their standard deviation, for the results obtained from repeating the assay in quadruplicate.

| Treatment | Lesion/Growth (mm) | Diameter | Inhibition percentage of *B. cinerea* (%) |
|---|---|---|---|
| *B. cinerea* | 8 ± 4 | | N.A. |
| RGM 2450 | 1 ± 0.3 | | N.A. |
| RGM 2529 | 2.3 ± 0.8 | | N.A. |
| Strain mixture | 2.6 ± 0.6 | | N.A. |
| QST 713 | 0.8 ± 0.5 | | N.A. |
| RGM 2450 vs. B. | 4 ± 1 *cinerea* | | 50 |

(continued)

| Treatment | Lesion/Growth (mm) | Diameter | Inhibition percentage of *B. cinerea* (%) |
|---|---|---|---|
| RGM 2529 vs. B. 4 $\pm$ 2 *cinerea* | | | 50 |
| Strain mixture 5 $\pm$ 2 vs. *B. cinerea* | | | 50 |
| QST 713 vs. B. 8 $\pm$ 3 *cinerea* | | | 0 |

The assays show significantly greater inhibition of phytopathogen growth by the RGM 2450 and RGM 2529 strains, compared to the commercial product devoid of its coformulants (*Bacillus subitillis* QST 713 bacteria only), achieving a 50% inhibition percentage by using each RGM strain separately, and by applying a mixture of both.

**Example 4. Field test of PGPR activity in strains *Bacillus safensis* RGM 2450 and *Bacillus siamensis* RGM 2529: greenhouse tomato culture**

The objective of this application example is to evaluate the effect of inoculating the strains *Bacillus safensis* RGM 2450 and *Bacillus siamensis* RGM 2529, separately and together, on productive parameters in tomato plants under greenhouse conditions.

In particular, the effect of formulations in the wettable powder, effervescent tablet and extended-release tablet delivery formats on strains RGM 2450 and RGM 2529 is evaluated. For the initial preparation of bacterial cultures of *B. safensis* RGM 2450 and *B. siamensis* RGM 2529, fermentation was carried out per batch fed in stirred tank bioreactors (Applikon (Applikon Biotechnology á) up to 5L of operation.

The obtained microbial concentrates were harvested and stored at 4°C in sterile 2L pyrex (Duraná) bottles. The concentrates were sampled monthly for 5 months maintaining their average viability at $10^9$ CFU/mL. These concentrates were used to inoculate seeds and prepare the different formulations: Extended-Release Tablet (ERT), Effervescent Tablet (ET) and Wettable Powder (WP). In addition, the different formulations were used in conjunction with different concentrations of extended-release fertilizers to determine the effect of bacteria on the tomato crop.

To facilitate understanding, the stages of the assay are presented sequentially, divided into the following phases: seed inoculation and transplant (4.1), differentiated fertilization (4.2), formulation preparation (5.3), application of formulations to plants (4.4), design of experiments (4.5), plant management (4.6), and determination of parameters of productive interest in tomato crops: determination of length, weight and tomatoes, and determination of chlorophyll (4.7). Finally, the results of the assay will be presented in point 4.8.

4.1. Seed inoculation and transplant.

The seeds used in this assay were of the Roma VF (VitaÓ) variety. Prior to inoculation, these were disinfected with 2% sodium hypochlorite for 3 min, and then washed 5 times with sterile distilled water to remove the disinfectant. All treatments were inoculated with bacteria from seed (with the exception of the control). The bacteria were washed of the coadjuvants that may exist in the culture medium in which they grew that could interact with the plant and produce altered results due to the presence of nutrients. Washes were performed 3 times with sterile PBS buffer (137 mM NaCl, 2.7 mM KCl, 10 mM Na2HPO4, 1.8 mM KH2PO4, pH = 7.2) together with subsequent centrifugation at 5000 rpm $\times$ 5 min, discarding the supernatant after each centrifugation. The pellets were resuspended in PBS buffer with both strains remaining at a concentration of $10^9$ CFU/mL.

Subsequently, the seeds were soaked in buffer with the bacteria for 40 min stirred at 200 rpm. For the commercial control, the seeds were put into solution at the same concentration as the strains of interest, $10^9$ CFU/mL. After inoculation, the seeds were superficially sown into a composition substrate of 2:1:1 peat, perlite and compost, respectively, in trays 110 mm deep and with 5 $\times$ 5 cm surface area in the greenhouse. Subsequently, the resulting seedlings were transplanted into 5L pots. The assay was performed in a 8 $\times$ 9 m (width $\times$ length) biosafety greenhouse with automatic irrigation and controlled temperature of 25°C$\pm$5.

4.2 Differentiated fertilization.

The assays used ANASAC Multicote Agri® fertilizer (12 m). It was added and homogenized with the substrate (described above). In addition, the fertilizer was added at different concentrations described in Table 12, to quantify the response (weight and caliber) of the fertilization-microorganism interaction.

**Table 12. Different fertilization percentages used for each plant. 100% fertilization corresponds to the application dose as indicated by the manufacturer.**

| % Fertilization | Grams of Multicote Agri® (12 m) / Plant |
|---|---|
| 0 | 0 |
| 33 | 13.2 |
| 66 | 26.4 |
| 100 | 40 |

4.3 Formulation preparation

The formulations were made according to Table 13, showing the ingredients and concentrations used for the preparation of the different proposed bacterial formulations. To determine masses and volumes, the density of the bacteria in the liquid culture medium (microbial broth) was considered to be equal to 1 g/mL. To reach the indicated CFU/g concentration of product, it was necessary to dilute or concentrate the bacteria, as required.
This was done in proportion to its ingredients, so that every 100g of formula had a concentration of $2*10^{10}$ CFU.
Wettable powder (WP), extended-release tablet (ERT) and effervescent tablet (ET) formulations were prepared.

**Table 13. Ingredients and concentrations for the preparation of the different proposed bacterial formulations.**

| Product | Ingredients | mg/g product | CFU/g of product |
|---|---|---|---|
| Wettable powder (WP) | Silicon dioxide | 150 | $10^8$ |
| | Potato starch | 170 | |
| | **bacteria** | 680 | |
| Extended-release tablet (ERT) | Wheat Flour | 280 | |
| | Corn starch | 280 | |
| | gelatin | 18 | |
| | Hot water (gelatin) | 140 | |
| | **bacteria** | 280 | |
| Effervescent tablet (ET) | Citric acid | 300 | |
| | Sodium bicarbonate | 660 | |
| | **bacteria** | 33 | |

4.4 Application of Formulations to plants

The application of the bacteria was carried out in the formulation formats already described: ERT, ET and WP. Applications were made at the base of the plant stem in the 5L pot.
The ET and WP formulations were dissolved in water to apply no more than 10 mL per pot, equivalent to $10^8$ CFU/plant (also for commercial control). The ERT was introduced into the substrate at the base of the plant, between the stem and automatic irrigation dropper, and its concentration was also equivalent to $10^8$ CFU/plant. Formulations were applied at 60 d from the start of culture.

4.5 Design of experiments

The experiment was designed with the formulations ERT, ET and WP, plus a commercial control and witness together with 4 doses of differentiated fertilization (Table 13), resulting in a total of 44 treatments (Table 14), with 3 replicates each, resulting in 132 pots in total. A single evaluation was performed at its production stage at the end of the culture. The assessments were the accumulated tomato weight at the seventh week of cultivation along with its caliber and the chlorophyll in the foliage.

**Table 14. Treatment table. Percentages correspond to fertilizations of Multicote Agri in combination with formulations (ERT, ET and WP) and bacteria RGM 2529 and RGM 2450, and their Mixture (1:1).**

| No. | Treatment | No. | Treatment |
|---|---|---|---|
| 1 | Control 0% | 23 | 2529 ERT 66% |
| 2 | Control 33% | 24 | 2529 ERT 100% |
| 3 | Control 66% | 25 | 2529 ET 0% |
| 4 | Control 100% | 26 | 2529 ET 33% |
| 5 | Commercial 0% | 27 | 2529 ET 66% |
| 6 | Commercial 33% | 28 | 2529 ET 100% |
| 7 | Commercial 66% | 29 | 2529 WP 0% |
| 8 | Commercial 100% | 30 | 2529 WP 33% |
| 9 | Mix ERT 0% | 31 | 2529 WP 66% |
| 10 | Mix ERT 33% | 32 | 2529 WP 100% |
| 11 | Mix ERT 66% | 33 | 2450 ERT 0% |
| 12 | Mix ERT 100% | 34 | 2450 ERT 33% |
| 13 | Mix ET 0% | 35 | 2450 ERT 66% |
| 14 | Mix ET 33% | 36 | 2450 ERT 100% |
| 15 | Mix ET 66% | 37 | 2450 ET 0% |
| 16 | Mix ET 100% | 38 | 2450 ET 33% |
| 17 | Mix WP 0% | 39 | 2450 ET 66% |
| 18 | Mix WP 33% | 40 | 2450 ET 100% |
| 19 | Mix WP 66% | 41 | 2450 WP 0% |
| 20 | Mix WP 100% | 42 | 2450 WP 33% |
| 21 | 2529 ERT 0% | 43 | 2450 WP 66% |
| 22 | 2529 ERT 33% | 44 | 2450 WP 100% |

4.6 Plant management

The plants were managed by performing the following applications and techniques:

1. Calcium application: due to the nutritional calcium requirements of the variety used. In total, 4 Ultrasol® Calcium applications were performed, adding 0.8g/plant in each application.

2. Apical cutting: at 100 days the apex of all plants was cut in order to slow vegetative growth and begin the development and maturation of fruits.

3. Debudding: All plants were debudded as these were detected. 4. Stripping: after 120 days of cultivation, all the leaves below the first bunch were cut with scissors, leaving only 1 leaf (the closest one) below it. This helps air flow between plants and improves fruit caliber.

4.7 Determination of parameters of productive interest in crops

Two general parameters of productive interest were determined or defined: 1. length, weight and tomatoes (number), and 2. concentration of chlorophyll. The protocols for each case are described below:

1. Determination of length, weight and tomatoes (number)

Tomatoes were evaluated by measuring their wet weight immediately after harvesting on a digital scale. The caliber was measured with a caliper at the longitudinal and equatorial diameter. The values were recorded on worksheets that were subsequently transferred to the database of cumulative weights and average diameters (equatorial and longitudinal). The results were tabulated and plotted as a surface response in Excel (Office 365) and statistically analyzed with the LSD test (a=0.05) in the program Statgraphic Centurion XVI (Statgraphics Technologies, Inc., USA).

2. Determination of chlorophyll

Chlorophyll was measured by removing the first three true leaves below the apex of each plant. From these, a segment was removed from the center of the leaf, attached to the central rib, not counting the latter, to complete 80 mg of leaves according to the methodology proposed by Wellburn (1994). For quantification of chlorophyll A and B: the collected leaves were frozen and stored at -20°C, then for each quantification, 0.5 mL of dimethyl sulfoxide (DMSO, EMSURE®) was added to 80 mg of leaves sprayed with liquid nitrogen in a microtube covered with aluminum foil. The solution was homogenized with a vortex. An additional 0.5 mL of DMSO was then added, and the mixture was incubated in a water bath at 65°C for two hours, followed by incubation at room temperature for 20 minutes. Finally, the content was centrifuged to avoid interference from suspended solids, and absorbance was measured using a spectrophotometer (Bioware WPA). The chlorophyll content was calculated using the following formulas (Wellburn 1994): Formula 1: Chlorophyll a ($\mu$g mL-1) = 12.47 * A665.1 - 3.62 * A649.1 Formula 2: Chlorophyll b ($\mu$g mL-1) = 25.06 * A649.1 - 6.5 * A665.1

4.8 <u>Assay results</u>

Treatments generally showed qualitative and quantitative effects.

Qualitatively, for the different treatments of the bacterial mixture, a robust pattern was observed with abundant leaf mass and smaller plant size. Fresh weight, dry weight and plant length were not evaluated in this assay because all treatments were debudded, stripped, and the main apex was removed to stop their apical growth and promote fruit maturation.

As for the caliber of the tomatoes, through the seventh week of harvest, calibers have no statistically significant difference between most treatments, differing only in some extreme treatments, e. g., Control vs Mixture. The average gauge is between 40 and 50 mm.

The parameter of cumulative tomato weight per treatment had statistically significant differences between most treatments through the seventh week of harvest. The treatment that had a statistically significant difference response compared to all other treatments was the Mix WP 66% fertilization, with a peak exceeding 900 g, far exceeding the control and commercial treatments (Figure 16).

Finally, when the concentration of chlorophyll ($\mu$g/mL) was determined at the seventh week of harvest, significant statistical differences were observed between most treatments. The treatment that had a statistically significant response different from all other treatments was the Mix ERT 100% fertilization, exceeding 250 $\mu$g/mL chlorophyll (Figure 17).

**Example 5. Study to Predict Antimicrobial Compounds and Enzymes of *Bacillus safensis* RGM 2450 and *Bacillus siamensis* RGM 2529**

The genomes of *Bacillus safensis* RGM 2450 and *Bacillus siamensis* RGM 2529 were sequenced, assembled and analyzed.

Bioinformatics analysis identified and predicted genes involved in the synthesis of different ribosomal and non-ribosomal peptides.

Table 15 presents the results obtained for *B. safensis* RGM 2450 regarding antimicrobial compounds that would be secreted by *B*. and potential white phytopathogens. Table 16 describes the potential enzymes it would secrete.

*B. safensis* RGM 2450 has the ability to produce antimicrobial compounds as microkines.

According to the analysis, when comparing the gene cluster of the RGM 2450 strain to the gene cluster of the B. pumilus ATCC 7061 and B. venelenzis FZB42 strains that are involved in the synthesis of plantazolicin, it is possible to determine that the RGM 2450 strain has 11 of the 12 genes of the pzn gene cluster that are involved in the synthesis and transport of the microkine plantazolicin (Fig. 18). This microkine has been described as having antibacterial activity against bacteria such as *Brevibacillus brevis, Bacillus cereus, Bacillus licheniformis, other Bacillus species, Micrococcus luteus, and Paenibacillus granivorans* (Table 15).

*B. safensis* RGM 2450 has the ability to produce the non-ribosomal peptide bacillibactin. According to the analysis of the RGM 2450 genome, the DhbACEBF gene cluster (Fig. 19A) was predicted to be involved in the synthesis and assembly of the bacillibactin siderophore along with genes involved in the transport (FeuABC-YusV) and hydrolysis of the siderophore for the release of Fe+3 (Fig 19B). Bacillibactine has previously been recognized for its inhibitory activity against the fungus *F. oxysporum f. sp. capsici* (Table 15).

The analysis also predicted the potential production of cyclolipopeptide. Particularly, a 46673 bp gene cluster consisting of 5 biosynthetic genes encoding modular proteins that make up a non-ribosomal peptide synthetase was predicted (Fig. 20A). Based on the bioinformatics prediction, the ribosomal peptide synthetase is encoded by 5 genes, of which gene 1 (10707 bp) encodes a multimodular protein that would be involved in the synthesis of a three amino acid peptide (leucine-leucine-glutamine), gene 2 (10701 bp) encodes the synthesis of a second module involved in the synthesis of a three amino acid peptide (leucine-aspartic acid- X (unable to predict)), gene 3 is involved in the addition of the amino acid isoleucine, gene 4 presents two modules that would allow the addition of the amino acid valine and an acetyl-CoA molecule, which could be involved in peptide cycling. Gene 5 presents two modules that are involved in the addition of another amino acid (X) and a fatty acid (Figs. 20B and 20C), a background that suggests that the potential structure that could be formed is a cyclolipopeptide.

Then, to define which molecules could correspond to this potential cyclolipopeptide that the RGM 2450 strain would produce, the search and comparison of the gene cluster in other sequenced strains was performed, observing a high degree of conservation (synthenia) in species *B. safensis* and *B. pumilus,* suggesting that it plays an important role in secondary metabolism (Fig. 21A). However, a portion of the genes shows homology with the cluster involved in the synthesis of the surfactant lichenysin, described in *B. licheniformis* DSM 13 (Fig. 21B).

When predicting the synthesis of the antibiotic bacilysin by bioinformatic analysis, the genes that were part of the *bacABCDE* gene cluster involved in its synthesis were identified (Fig. 22A). This compound is a dipeptide antibiotic consisting of an L-alanine residue that is bound to a non-proteinogenic amino acid, L-anticapsin (Fig 22B). The gene cluster that participates in the synthesis of bacilysin as its gene neighborhood are highly conserved in *Bacillus* and phylogenetically close to *B. safensis* (Fig. 22C).

In addition, *B. safensis* RGM 2450 can potentially secrete various enzymes such as proteases, metalloproteases and lipases, among others (Table 16).

**Table 15. Compounds that would be secreted by *B. safensis* RGM 2450 and potential white phytopathogens.**

| Compound | Antibacterial activity | Antifungal activity | Reference |
|---|---|---|---|
| Plantazolicin (microkine) | *Brevibacillus brevis, Bacillus cereus, Bacillus licheniformis, Bacillus megaterium, Bacillus pumilus, Bacillus sphaericus, B. subtilis, Micrococcus luteus, Paenibacillus granivorans.* | | Scholz et al., 2011 |
| Bacillibactin (siderophore) | - | *F. oxysporum f sp. capsici* | Yu et al., 2011 |
| Bacilysin (dipeptide) | *Clavibacte michiganense subsp. sepedonicum, Erwinia amylovora, Escherichia coli, Salmonella typhi, Staphylococcus aureus, Streptococcus pyogenes* | *Candida albicans, Microcystis aeruginosa, phytophthora infestans, S. cerevisiae, Aspergillus fumigatus, Candida albicans* | Kenig et al., 1976; Kenig and Abraham, 1976; Loeffler et al., 1986; Zuber et al., 1993; Steinborn et al., 2005; Chen et al., 2009; Wu et al., 2014; Wu et al., 2015a; Caulier et al., 2018 |

**Table 16. Potential enzymes that would be secreted by *B. safensis* RGM 2450.**

| Extracellular enzymes |
|---|
| Protease, alkaline protease |
| 2 proteases, subtilisin type E |
| Protease, Bacillopeptidase F (EC 3.4.21.-) |
| Protease, metalloprotease |
| Protease, Serine protease AprX |
| Lipase, lipase (EC 3.1.1.3) |
| Lactonase, YtnP Lactonase |

For its part, *B. siamensis* RGM 2529 would secrete bacteriocins such as amylocyclicin. Based on the genome analysis of the RGM 2529 strain, the 4170 bp acnABCDEF gene cluster that would be involved in the synthesis, maturation and self-protection of the antimicrobial peptide (bacteriocin) amylocyclicin was predicted (Fig. 23A). The acnABCDEF gene cluster encoding aminocyclin has been found in strains of the species *Bacillus amyloliquefaciens* and *B. velezensis,* which has been widely described for its PGPR activity (Fig. 23B). Amylocyclicin has been described in the literature as having antibacterial activity against *Brevibacillus brevis, Bacillus cereus, Bacillus licheniformis, Bacillus megaterium, B. pumilus, Bacillus sphaericus, B. subtilis, Clavibacter michiganensis, Micrococcus luteus, Paenibacillus granivorans, and Paenibacillus polymyxa* (Table 17)

From the analyses, it is demonstrated that *B. siamensis* RGM 2529 presents the DhbACEBF gene cluster that is involved in the synthesis of bacillibactin (Figure 24), a siderophore with antifungal activity against *F. oxysporumf. sp. capsici* (Table 17). It also presents the 4 genes (*surfAA, surfAB, surfAC,* and *surf*AD) that are involved in the biosynthesis and assembly of the amino acids that make up surfactin (Figure 25A). Based on the analysis of the surfactin gene cluster in strains phylogenetically close to the RGM 2529 strain (Figure 25B), comparing the *surf*AB homolog of *B. velezensis* ZB42 with that of RGM 2529 yields 94.95% identity and 73% coverage. Meanwhile, the surfAC gene encodes a ribosomal peptide synthetase consisting of one module involved in leucine assembly. Comparing the surfAC homolog of *B. amyloliquefaciens* ZB42 to that of RGM 2529 yields 94.95% identity and 100% coverage. Finally, the surfAD gene encodes a thioesterase. Comparing the surfAD homolog of *B. velezensis* ZB42 with that of RGM 2529 yields 95.36% identity and 100% coverage.

For fengycin, a compound previously described as antifungal, a 49465 bp gene cluster was also predicted to be involved in the synthesis of this lipopeptide. This gene cluster is made up of 5 genes (*fen*A, *fenB, fenC, fenD,* and *fenF*) that encode the non-ribosomal peptide synthetases that assemble this cyclolipopeptide along with other genes involved in its structural modification (Fig. 25A). This gene cluster has been found in strains belonging to the species *B. amyloliquefaciens, B. velezensis* and B. siamensis (Fig. 25B). The assembly of amino acids by synthetases gives rise to a cyclodecapeptide that, together with the action of the fatty acid enzyme ligase (long chain fatty acid CoA ligase), form a cyclolipopeptide (Figure 26C).

Adjacent to the gene cluster involved in the synthesis of fengycin, the operon bamABCD involved in the synthesis of another lipopeptide, bacillomycin D, was found (Fig. 27A). This operon covers 37696 bp and has been described in species B. *amyloliquefaciens, B. velezensis* and *B. siamensis* (Fig. 27B).

It has been described that bacillomycin D can remove fungi from different genus and species, including *Alternaría alternata, Alternaría solani, Botrytis cinerea, Aspergillus flavus' Botryosphaerica ribis, C. albicans, Cryphonectria parasítica, Colletotrichum acutatum, Colletotrichum gloesporioides, Didymella bryoniae, Fusarium graminearum, Fusarium sambucinum, Fusarium oxysporum, Podosphaera fusca, Pythium sulcatum, Pythium ultimum, Rhizoctonia solani, Rhizopus* sp., *Sclerotinia sclerotiorum, Ustilago maydis, Monilinia fructicola, Penicillium expansum, Phomopsis gossypii, Phytophthora capsici, Pyricularia grisea, R. solani, Sclerotium rolfsii, and Sclerotinia sclerotiorum* (Table 17).

A 72346 bp gene cluster (Fig. 28A) encoding a hybrid between a polyketide synthase and a non-ribosomal peptide synthetase (PKS-NRPS) involved in the production of the antibiotic bacillaene, an inhibitor of prokaryotic protein synthesis, was also predicted. The bacillaene synthase of the RGM 2529 strain consists of 13 PKS modules and 3 NRPS modules, as described in *velezensis* FZB42 and *Bacillus subtilis* 168 (Fig. 28B).

Therefore, *B. siamensis* RGM 2529 can potentially produce the antibacterial and antifungal compound bacillaene, for which it has been described as having potential for action against bacteria such as *B. thuringiensis, E. coli, Klebsiella pneumoniae, M. xanthus, P. vulgaris, Serratia marcescens, and S. aureusy. In the case of fungi, it has been described that bacillaene has inhibitory activity against Coriolopsis spp., Fusarium sp, Pseudoxylaria sp., Trichoderma sp., and Umbelopsis sp.* (Table 17).

Finally, *B. siamensis* RGM 2529 would also be able to secrete protease, lipase, lactonase, and cellulase enzymes (Table 18).

**Table 17. Compounds that would be secreted by *B. siamensis* RGM 2529 and potential white phytopathogens.**

| Compound | Antibacterial activity | Antifungal activity | Reference |
|---|---|---|---|
| Amylocyclicin (bacteriocin) | *Brevibacillus brevis, Bacillus cereus, Bacillus licheniformis, Bacillus megaterium, B. pumilus, Bacillus sphaericus, B. subtilis, Clavibacter michiganensis, Micrococcus luteus, Paenibacillus granivorans, Paenibacillus polymyxa* | | Scholz *et al.,* 2014 |

(continued)

| Compound | Antibacterial activity | Antifungal activity | Reference |
|---|---|---|---|
| Fengycin (lipopeptide) | - | *Alternaría solani, Botrytis cinerea,* | Cawoy *et al.,* 2014; Ongena *et al.,* 2005; |
| | | *Fusarium graminearum, Fusarium sambucinum, Fusarium oxysporum, Podosphaera fusca, Pythium sulcatum, Pythium ultimum, Rhizoctonia solani, Rhizopus* sp., *Sclerotinia sclerotiorum* | Ramarathnam *et al.,* 2007; Romero *et al.,* 2007; Guo et al., 2014; Wise *et al.,* 2014; Zhao *et al.,* 2014 |
| Bacillomycin D (lipopeptide) | - | *Alternaría alternata, A. solani, Aspergillus flavus, Botryosphaerica ribis, C. albicans, Cryphonectria parasitica, Colletotrichum acutatum, Colletotrichum gloesporioides, Didymella bryoniae, F. graminearum, F. oxysporum, Ustilago maydis, Monilinia fructicola, Penicillium expansum, Phomopsis gossypii, Phytophthora capsici, Pyricularia grisea, R. solani, Sclerotium rolfsii, S. sclerotiorum* | Moyne *et al.* 2001; Gong *et al.,* 2014; Zhao *et al.,* 2010; Yuan *et al.,* 2012; Tanaka *et al.,* 2014 |
| *Surfactin (lipopeptide)* | *L. pneumophila, L. monocytogenes, P. syringae, R. solanacearum, S. aureus, Xanthomonas axonopodis pv. glycines* | *A. niger, B. cinerea, F. oxysporum, F. solani, Monilia fructigena, Pennicilium expansum, P. italicum, R. solani* | *Bais et al., 2004; Cawoy et al., 2014; Preecha et al., 2010; Gao L. et al., 2017; Romano et al., 2013; Sabaté and Audisio, 2013; Kwon and Kim, 2014; Loiseau et al., 2015; Luo et al., 2014* |
| *Bacillibactin (siderophore)* | | *F. oxysporum f. sp. capsici* | *Yu et al., 2011* |
| *Bacillaene (polyene)* | *B. thuringiensis, E. coli, Klebsiella pneumoniae, M. xanthus, P. vulgaris, Serratia marcescens, S. aureus* | *Choriolopsis spp., Fusarium sp, Pseudoxylaria sp., Trichoderma sp., Umbelopsis sp.* | *Patel et al., 1995; Um et al., 2013; Müller et al., 2014* |
| *Aurantinin* | *S. aureus ATCC 25923, M. luteus CMCC 28001, B. pumilus CMCC 63202, B. cereus ATCC 14579, B. subtilis ATCC 168, Listeria monocytogenes CICC 21662, Enterococcus faecalis ATCC 29212, and P. fluorescens ATCC 49642* | | *Couler et al., 2019* |

**Table 18. Potential enzymes that would be secreted by *B. siamensis RGM 2529*.**

| **Extracellular enzymes** |
|---|
| Protease, Serine protease AprX |
| Protease, Subtilisin E (EC 3.4.21.62) |

(continued)

| Extracellular enzymes |
| --- |
| Protease, Bacillopeptidase F (EC 3.4.21.-) |
| Protease, Bacillolysin, Metalloprotease (EC 3.4.24.28) |
| Lipase, Lipase (EC 3.1.1.3) |
| Lactonase, YtnP Lactonase |
| Cellulase, beta-1,4-glucanase (EC 3.2.1.4) |

**Literature:**

Mayak S., Tirosh T., Glick B. R. 2004. Plant growth promoting bacteria that confer resistance to water stress in tomato and pepper. Plant Sci. 166; 525-530.

Criollo P, Obando M, Sanchez L, Bonilla R. (2012) Effect of plant growth-promoting rhizobacteria (PGPR) associated to Pennisetum clandestinum in the highlands of Cundiboyacense. Slurry Corpoica, Vol. Agriculture Technology 13 No. 2

Altier N, Beyhaut E, Dalla M, Rivas F. (2012). Environmental Sustainability: Platform for bio-inputs for agricultural use based on beneficial microorganisms. INIA Uruguay Magazine No. 29, 47-50.

Hayat R, Ali S, Amara U, Khalid R, Ahmed I (2010) Soil beneficial bacteria and their role in plant growth promotion: a review. Ann Microbiol 60(4), 579-598.

Etesami H, Emami S, Alikhani HA. (2017). Potassium solubilizing bacteria (KSB): Mechanisms, promotion of plant growth, and future prospects - A review. Journal of soil science and plant nutrition, 17: 897-911

Yadav S., Kaushik R., Saxena A.K. & Arora D.K. 2011. Genetic and functional diversity of Bacillus strains in the soils longterm irrigated with paper and pulp mill effluent. J. Gen. Appl. Microbiol. 57: 183-195.

Chakraborty U., Chakraborty B.N., Chakraborty A.P. & Dey P.L. 2013. Water stress amelioration and plant growth promotion in wheat plants by osmotic stress tolerant bacteria. World J. Microbiol Biotechnol. 29: 789-803.

Kavamura V.N., Santos S.N., Silva J.L., Parma M.M., Avila L.A., Visconti A., Zucchi T.D., Taketani R.G., Andreote F.D. & Melo I.S. 2013. Screening of Brazilian cacti rhizobacteria for plant growth promotion under drought. Microbiol. Res. 168: 183-191

Sun H., He Y., Xiao Q., Ye R. & Tian Y. 2013. Isolation, characterization, and antimicrobial activity of endophytic bacteria from Polygonum cuspidatum. Afr. J. Microbiol. Res. 7 1496-1504

Jeong H, Jeong DE, Kim SH, Song GC, Park SY, Ryu CM, Park SH, Choi SK. 2012.Draft Genome Sequence of the Plant Growth-Promoting Bacterium Bacillus siamensis KCTC 13613. J Bacteriol. 194:4148-4149. doi: 10.1128/JB.00805-12.

Wu, L., Wu, H. J., Qiao, J., Gao, X., and Borriss, R. (2015). Novel routes for improving biocontrol activity of Bacillus based bioinoculants. Front. Microbiol. 6:1395.

Haeyoung Jeong, Da-Eun Jeong, Sun Hong Kim, Geun Cheol Song, Soo-Young Park, Choong-Min Ryu, Seung-Hwan Parkand Soo-Keun Choi (2012). Draft Genome Sequence of the Plant Growth-Promoting Bacterium Bacillus siamensis KCTC 13613. J Bacteriol. 194(15): 4148-4149.

Borriss, R. (2011). "Use of plant-associated Bacillus strains as biofertilizers and biocontrol agents," in Bacteria in Agrobiology: Plant Growth Response, ed D. K. Maheshwari (Heidelberg: Springer), 41-76.

Agbaje Lateef, Isiaka Adedayo Adelere and Evariste Bosco Gueguim-Kana. (2015). The biology and potential biotechnological applications of Bacillus safensis. Biology 70/4: 411-419, 2015 Section Cellular and Molecular Biology.

**Claims**

1. An agricultural formulation to promote growth and/or increase crop yield and/or protect against diseases and pests **CHARACTERIZED by** comprising at least:

    a) One bacterial strain *Bacillus safensis* deposit RGM 2450, and/or
    b) One bacterial strain Bacillus *siamensis* deposit RGM 2529, and
    c) Excipients for agricultural use.

2. An agricultural formulation for promoting growth and/or increasing crop yield and/or protecting against diseases and pests according to claim 1 **CHARACTERIZED by** the bacterial strain *Bacillus siamensis* deposit RGM 2529 having a concentration or amount of $3.0 \times 10^6$ - $4.0 \times 10^9$ CFU/g in the formulation.

3. An agricultural formulation for promoting growth and/or increasing crop yield and/or protecting against diseases and pests according to claim 1 **CHARACTERIZED by** the bacterial strain *Bacillus safensis* deposit RGM 2450 having a concentration or amount of $5.0 \times 10^6$ - $4 \times 10^9$ CFU/g in the formulation.

4. An agricultural formulation to promote growth and/or increase crop yield and/or protect against diseases and pests according to claims 1-3 **CHARACTERIZED by** excipients for agricultural use corresponding to, but not being limited to: wheat flour, corn starch, gelatin, potato starch, silicon dioxide, citric acid, bicarbonate, polysorbates like Tweens, lactose, soy lecithin, casein, carboxymethyl cellulose or cellulose gum, sucrose esters, mannitol, sorbitans, Pluronic F68, alginate, xanthan gum, PEG (polyethylene glycol), corn syrup, egg, milk, glycerol, fructose, pectins, mineral oil, ester gum, long chain triglycerides.

5. An agricultural formulation for promoting growth and/or increasing crop yield and/or protecting against diseases and pests according to claims 1-4 **CHARACTERIZED by** the formulation being presented as an extended-release tablet.

6. An agricultural formulation for promoting growth and/or increasing crop yield and/or protecting against diseases and pests according to claims 1-4 **CHARACTERIZED by** the formulation being presented as a wettable powder.

7. An agricultural formulation for promoting growth and/or increasing crop yield and/or protecting against diseases and pests according to claims 1-4 **CHARACTERIZED by** the formulation being presented as an effervescent tablet.

8. An agricultural formulation for promoting growth and/or increasing crop yield and/or protecting against diseases and pests according to claims 1-7 **CHARACTERIZED by** the formulation being presented as a resuspension or emulsion of bacterial strains or a mixture of these.

9. Use of an agricultural formulation according to claims 1-8 **CHARACTERIZED by** said formulation serving to increase the nitrogen binding of a crop.

10. Use of an agricultural formulation according to claims 1-8 **CHARACTERIZED by** said formulation serving to increase the solubilization of phosphorus and potassium, and phytohormone production capacity of a crop.

11. Use of an agricultural formulation according to claims 1-8 **CHARACTERIZED by** said formulation serving as a pathogen control agent or biocontroller in a crop.

12. Use of an agricultural formulation according to claim 11 **CHARACTERIZED by** said formulation serving as a bioprotective agent and/or biocontroller for crops against pathogens such as *Fusarum* sp., *Phytophthora* sp., *Collectotrichum* sp., and *Botrytis* sp.

13. Use of an agricultural formulation according to claims 1-8 **CHARACTERIZED by** said formulation serving as a bioprotective agent and/or biocontroller for crops against pathogens such as *S. aureus ATCC 25923, M. luteus CMCC 28001, B. pumilus CMCC 63202, B. cereus ATCC 14579, B. subtilis ATCC 168, L. monocytogenes CICC 21662, E. faecalis ATCC 29212 and P. fluorescens ATCC 49642, B. thuringiensis, Klebsiella pneumoniae, M.*

*xanthus, P. vulgaris, Serratia marcescens, S. aureus, L. pneumophila, L. monocytogenes, P. syringae, A. solanacearum, Xanthomonas axonopodis pv. Glycines, B. brevis, B. cereus, B. licheniformis, B. megaterium, B. pumilus, B. sphaericus, B. subtilis, C. michiganensis, M. luteus, P. granivorans, P. polymyxa, Coriolopsis spp. Fusarium sp, Pseudoxylaria sp., Trichoderma sp., Umbelopsis sp., F. oxysporum f. sp., Capsici, A. niger, F. solani, M. fructigena, Pennicilium expansum, P. italicum, P. solani, Alternaria alternata, A. solani, Aspergillus flavus, Botryosphaerica ribis, C. albicans, C. parasitica, C. acutatum, C. gloesporioides, Didymella bryoniae, F. graminearum, F. oxysporum, Ustilago maydis, Monilinia fructicola, P. expansum, P. gossypii, P. capsici, P. grisea, P. solani, S. rolfsii, S. sclerotiorum, A. solani, B. cinerea, F. graminearum, F. sambucinum, F. oxysporum, P. fusca, P. sulcatum, P. ultimum, P. solani, Rhizopus sp., S. sclerotiorum, C. michiganense subsp. sepedonicum, E. amylovora, E. coli, S. typhi, S. pyogenes, C. albicans, M. aeruginosa, P. infestans, S. cerevisiae, A. fumigatus.*

14. Use of an agricultural formulation according to claims 1-8 **CHARACTERIZED by** said formulation serving as an agent for increasing the concentration of chlorophyll in a crop or plant.

15. Use of an agricultural formulation according to claims 1-8 **CHARACTERIZED by** said formulation serving as an agent for increasing crop growth and/or yield.

16. Method of promoting crop growth and/or increasing yield and/or protecting against diseases and pests CHARACTERIZED as consisting of applying an effective amount of the agricultural formulation described in claims 1-8 to a crop.

17. Method for promoting crop growth and/or increasing yield and/or protecting against diseases and pests according to claim 16 CHARACTERIZED as consisting of applying the agricultural formulation in the form of resuspension, emulsion, effervescent tablet, extended-release tablet, wettable powder, or mixtures thereof.

18. Method for promoting crop growth and/or increasing yield and/or protecting against diseases and pests according to claims 16 and 17 **CHARACTERIZED by** its application through sprinkling or direct immersion of the formulation on the seed of the target crop.

19. Method for promoting crop growth and/or increasing yield and/or protecting against diseases and pests according to claim 18 **CHARACTERIZED by** the seed being impregnated in a buffer and fully submerged in a formulation comprising a concentration of $10^9$ CFU/mL of the bacteria mixture.

20. Method for promoting crop growth and/or increasing yield and/or protecting against diseases and pests according to claims 16 and 17 **CHARACTERIZED by** being applied to the base of the plants' stem.

21. Method for promoting crop growth and/or increasing yield and/or protecting against diseases and pests according to claim 20 **CHARACTERIZED by** being applied to the base of the plants' stem at a concentration of $10^8$ CFU/mL.

22. Method for promoting crop growth and/or increasing yield and/or protecting against diseases and pests according to claims 16-21 **CHARACTERIZED by** the formulation being potentially administered in conjunction with fertilizers.

● 2450
KC346446.1 Bacillus altitudinis 41KF2b[T]
AJ831844.2 Bacillus aerophilus strain 28K[T]
AJ831841.2 Bacillus stratosphericus 41KF2a[T]
KC346445.1 Bacillus pumilus DSM27[T]
KC346444.1 Bacillus safensis FO-036b[T]
● 2529
LHCC01000002 Bacillus velezensis KCTC 13012[T]
NZ JTKJ02000000 Bacillus methylotrophicus KACC 13105[T]
X60605.1 Bacillus amyloliquefaciens ATCC 23350[T]
NZ JTKJ02000000 Bacillus velezensisKACC 13105[T]
X68416.1 B.licheniformis DSM 13[T]
AJ831843.1 Bacillus aerius 24K[T]
AF302118.1 Bacillus sonorensis NRRL B-23154[T]
AJ583158.1 Bacillus indicus Sd/3[T]
D16273.1 Bacillus megaterium IAM 13418[T]
AB021185.1 Bacillus flexus IFO 15715[T]
AF526913.3 Bacillus odysseyi NRR B-30641[T]
AY505499.1 Bacillus aquimaris DSM 16205[T]
AF483624.1 Bacillus marisflavi DSM 16204[T]
D16272.1 Bacillus lentus IAM 12466[T]
AF234863.1 Bacillus nealsonii DSM 15077[T]
D16268.1 Bacillus firmus IAM 12464.[T]
X64465.1 B.methanolicus NCIMB 13113[T]
D78310.1 Bacillus badius ATCC 14574[T]
AJ542508.1 Bacillus bataviensis LMG 21833[T]
AJ606700.1 Bacillus arsenicus MTCC 4380[T]
AJ536198.1 Micrococcus luteus DSM 20030[T]

*B. Pumilus* Group

*B. amyloliquefaciens* Group

0.02

Figure 1

Figure 2

Figure 3

Figure 4

Figure 5

Figure 6

Figure 7

Figure 7 (continuation)

**A**

**B**

Figure 8

Figure 8 (continuation)

Figure 9

Figure 9 (continuation)

Figure 10

Figure 10 (continuation)

Figure 11

Figure 12

Figure 13

Figure 14

Figure 14 (continuation)

Figure 15

Figure 16

Figure 17

Figure 18

A)

Bacillibactin synthase component F, biosynthesis of NRPS (EC 2.7.7-), DhbF.
Isochorismatase (EC 3.3.2.1), DhbB.
Bacillibactin synthase component F, biosynthesis of NRPS(EC.2.7.7.58), DhbE.
Isochorismate synthase (EC 5.4.4.2), DhbC.
2,3-dihydroxybenzoate dehydrogenase (EC1.3.1.28), DhbA.
Trilacton hydrolase, BesA.
Other genes

B)

Figure 19

a)

b)

c)

Figure 20

**A)**

*B. safensis* RGM 2450

*Bacillus* sp. Root920

*B. safensis* BRM1

*B. pumilus* GLB197

*B. pumilus* TUAT1

*B. pumilus* B4134

*B. pumilus* GR-8

*Bacillus* sp. AM 13

*B. pumilus* B4133

*B. pumilus* SAFR-032

*B. pumilus* W3

**B)**

*B. safensis* RGM 2450

*Bacillus licheniformis* DSM 13 = ATCC 14580

Figure 21

A)

| | |
|---|---|
| Aspartate aminotransferase (EC 2.6.1.1), YwfG | Oxidoreductase, BacB |
| Alanine--anticapsin ligase (EC 6.3.2.49), BacD | Oxopropanoate isomerase (EC 5.3.3.19), BacA |
| Oxidoreductase, BacC | Transporter, YwfA |

B)

C)

B. safensis RGM 2450

Bacilysin

Bacillus sp. WP8

B. pumilus MTCC B6033

B. pumilus GLB197

B. pumilus W3

B. cellulasensis NIO-1130

B. pumilus NJ-M2

B. pumilus LK123

B. pumilus TUAT1

B. altitudes 41KF2b

B. safensis RIT372

Figure 22

A)

Amylocyclicin          Bacillibactin

*acnB, transmembrane* protein (essential for peptide synthesis)
*acnA,* circular bacteriocin precursor, essential for synthesis
*acnC,* processing, maturation and autoimmunity
*acnD,* ATP-binding subunit of the ABC transport type
*acnE,* ABC type transport accessory factor
*acnF,* immunity

B)

*Bacillus velezensis* GFP-2     Amylocyclicin

*Bacillus amyloliquefaciens* JJC33M

*Bacillus amyloliquefaciens* MBE1283

*Bacillus amyloliquefaciens* Y14

*Bacillus velezensis* CGMCC 11640

*Bacillus velezensis* Lzh-a42

*Bacillus velezensis* CN026

Figure 23

54

Figure 24

**A)**

| | |
|---|---|
| *yckJ* | *yciC*, Zinc binding GTPase |
| *yckI* | *yx01*, alcohol deshidrogenasa |
| Integral membrane protein involved in non-ribosomal peptide synthesis. | *yckC* |
| Phosphopanthenyl transferase involved in the synthesis of non-ribosomal peptide. | *yckD* |
| Aminotransferase | *nin* |
| *srf*AD, surfactin synthetase | *nuc*A |
| *srf*AC, surfactin synthetase | *hxl*B, 6-phospho-3-hexulose isomerase (EC 5.3.1.2) |
| *srf*AB, surfactin synthetase | *hxl*R, transcriptional regulator |
| *srf*AA, surfactin synthetase | *hxl*A, D-arabino-3-hexulose-formaldehyde lyase |

**B)**

*Bacillus velezensis* FZB42

*Bacillus velezensis* J01

*Bacillus siamensis* SRCM100169

*Bacillus siamensis* XY18

*Bacillus* sp. JFL15

*Bacillus siamensis* SCSIO 05746

*Bacillus velezensis* 9912D

*Bacillus amyloliquefaciens* H57

*Bacillus velezensis* CN026

*Bacillus* sp. Co1-6

Figure 25

A)

| | |
|---|---|
| *dacC*, D-alanyl-D-alanine carboxypeptidase (EC 3.4.16.4) | *yng*K |
| *fen*A, fengicin synthetase A | *yng*J, isovaleryl-CoA dehydrogenase (EC 1.3.8.4) |
| *fen*B, fengicin synthetase B | *yng*I, acetyl-CoA long fatty acid chain ligase (EC 6.2.1.3) |
| *fen*C, fengicin synthetase C | *yng*H, biotin carboxylase |
| *fen*D, fengicin synthetase D | *yng*G, hydroxymethylglutaryl-CoA lyase |
| *fen*E, fengicin synthetase E | *yng*F, methylglutaconyl-CoA lyase (EC 4.1.3.4) |
| *yng*L | *yng*E, methylcrotonyl-CoA carboxyl transferase (EC 6.4.1.4) |

B)

*Bacillus amyloliquefaciens* subsp. *plantarum* NAU-B3

*Bacillus amyloliquefaciens* UMAF6614

*Bacillus siamensis* SCSIO 05746

*Bacillus velezensis* 10075

*Bacillus amyloliquefaciens* B15

*Bacillus velezensis* Lzh-a42

C)

Figure 26

**A)**

Fengycin                                         Bacillomycin D

- *yxjC*, D-beta-hydroxybutyrate permease
- *scoB*, succinyl-CoA: 3-ketoacid-Coenzyme A transferase
- *scoA*, succinyl-CoA: 3-ketoacid-Coenzyme A transferase
- 3-Hydroxybutyrate dehydrogenase (EC 1.1.1.30)
- malonyl CoA acyltransacylase (EC 2.3.1.39)
- *bam*A, bacillomycin D synthetase A
- *bam*B, bacillomycin D synthetase B
- *bam*C, bacillomycin D synthetase C
- *ynfF*, arabinofuranosidase
- *xynD*, feraxan endoxylanase

**B)**

*Bacillus amyloliquefaciens* subsp. *plantarum* NAU-B3

*Bacillus amyloliquefaciens* UMAF6614

*Bacillus siamensis* SCSIO 05746

*Bacillus velezensis* 10075

*Bacillus amyloliquefaciens* B15

*Bacillus velezensis* Lzh-a42

Figure 27

**A)**

baeS, cytochrome P450 hydroxylase
baeR, polyketide synthase I
baeN, PKS-NRPS hybrid
baeM, polyketide synthase I
baeL, polyketide synthase I
baeJ, PKS-NRPS hybrid
baeI, enoyl-CoA hydratase

baeH, enoyl-CoA hydratase
baeG, 3-Hydroxy-3-methylglutaryl CoA synthase
acpK, acyl carrier protein
baeC, malonyl CoA acyltransferase transporter protein (EC 2.3.1.39)
baeD, malonyl CoA acyltransferase transporter protein (EC 2.3.1.39)
baeE, malonyl CoA acyltransferase transporter protein (EC 2.3.1.39)
baeB, hydroxyacylglutathione hydrolase

**B)**

Bacillus amyloliquefaciens CMW1

Bacillus velezensis D2-2

Bacillus velezensis M75

Bacillus sp. Co1-6

Bacillus siamensis SRCM100169

Bacillus amyloliquefaciens LM2303

Bacillus velezensis M27

Figure 28

**INTERNATIONAL SEARCH REPORT**

| International application No. |
| --- |
| PCT/CL2020/050169 |

| A. | CLASSIFICATION OF SUBJECT MATTER |
| --- | --- |
| | CIP: A01N63/22, C12N1/20, C12R1/07, C05G3/00, C05F11/08 (2021.01) |
| | According to International Patent Classification (IPC) or to both national classification and IPC |

| B. | FIELDS SEARCHED |
| --- | --- |
| Minimum documentation searched (classification system followed by classification symbols) |
| | CIP: A01N63/22, C12N1/20, C12R1/07, C05G3/00, C05F11/08 |
| Documentation searched other than minimum documentation to the extent that such documents are included in the fields searched |
| Electronic data base consulted during the international search (name of data base and, where practicable, search terms used) |
| | INAPI, STN, PATENTSCOPE, ESPACENET, GOOGLE |

| C. | DOCUMENTS CONSIDERED TO BE RELEVANT | |
| --- | --- | --- |
| Category* | Citation of document, with indication, where appropriate, of the relevant passages | Relevant to claim No. |
| X<br>Y | LATEEF, A. et al. The biology and potential biotechnological applications of Bacillus safensis. Biologia, 2015, vol. 70, no 4, p. 411-419. DOI: 10.1515/biolog-2015-0062<br>The whole document | 1, 4, 8, 11-13, 15-17<br>1-22 |
| X<br>Y | CN109456915 (HENAN AGRICULTURAL UNIVERSITY) 12-03-2019<br>The whole document | 1, 3, 4, 8-10, 15-17, 1-22 |
| X<br>Y | NGO, V., et al. Phytophthora antagonism of endophytic bacteria isolated from roots of black pepper (Piper nigrum L.). Agronomy, 2020, vol. 10, no 2, p. 286. DOI: 10.3390/agronomy10020286<br>The whole document | 1, 2, 4-8, 11-22, 1-22 |

| [X] Further documents are listed in the continuation of Box C. | [X] See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 31 May 2021 (31.05.2021) | 10 August 2021 (10.08.2021) |

| Name and mailing address of the ISA/ CL | Authorized officer |
| --- | --- |
| Facsimile No. | Telephone No. |

Form PCT/ISA/210 (second sheet) (January 2015)

| INTERNATIONAL SEARCH REPORT | International application No. |
|---|---|
| | PCT/CL2020/050169 |

C (Continuation). DOCUMENTS CONSIDERED TO BE RELEVANT

| Category* | Citation of document, with indication, where appropriate, of the relevant passages | Relevant to claim No. |
|---|---|---|
| A | CN109468243A (UNIV HEBEI AGRICULTURE) 15-03-2019. The whole document | |
| A | ZHANG, X., et al. Control effects of Bacillus siamensis G-3 volatile compounds on raspberry postharvest diseases caused by Botrytis cinerea and Rhizopus stolonifer. Biological Control, 2020, vol. 141, p. 104135. DOI:10.1016/j.biocontrol.2019.104135 The whole document | |

Form PCT/ISA/210 (continuation of second sheet) (January 2015)

**INTERNATIONAL SEARCH REPORT**

| International application No. |
| --- |
| PCT/CL2020/050169 |

---

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:

Claims 1 to 22 lack unity of invention. Claims 1 to 8 describe agricultural formulations comprising individual strains or a mixture of strains. The two strains are identified by their deposit number and by the number of species according to genetic studies carried out (table 2, page 22). It is concluded that the strains indicated in claims 1-8 are strains that are genetically different from each other.

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (January 2015)

INTERNATIONAL SEARCH REPORT

International application No.

PCT/CL2020/050169

(Supplemental sheet 1/1, continuation of Box III)

The solution to the technical problem is to produce agrochemical formulations comprising Bacillus siamensis and Bacillus safensis strains, either individually or combined, for controlling pathogens and/or for stimulating plant growth. Formulations comprising B. safensis (documents D1 and D2) or formulations comprising B. siamensis (D3) for stimulating the growth and/or increasing the yield of crops and/or protecting same from disease and pests are already known in the prior art. The prior art documents show empirical evidence that the B. safensis and B. siamensis strains exhibit antimicrobial activity and/or stimulate plant growth. Therefore, the common concept of claims 1-22 relating to agrochemical formulations comprising B. safensis and/or B. siamensis exhibiting antimicrobial activity and/or stimulating plant growth is known in the prior art and therefore breaks the unity of invention. Thus, formulations that comprise B. safensis and B. siamensis strains, individually or combined, are considered to be different inventions. Taking the above-mentioned into consideration, three groups of inventions can be discerned:

Group 1 (claim 1 in part, claim 3 in full, and claims 4-22 in part): Agricultural formulation comprising the Bacillus safensis strain RGM deposit number 2450 (CChRGM: the Chilean Collection of Microbial Genetic Resources), uses of said formulation, and methods for stimulating growth and/or increasing the yield of crops and/or protecting same from disease and pests, comprising said formulation.

Group 2 (claim 1 in part, claim 2 in full, claims 4-22 in part): Agricultural formulation comprising the Bacillus siamensis strain RGM deposit number 2529, uses of said formulation, and methods for stimulating growth and/or increasing the yield of crops and/or protecting same from disease and pests, comprising said formulation.

Group 3 (claims 1-22 in part): Agricultural formulation comprising the Bacillus safensis strain RGM deposit number 2450 and the Bacillus siamensis strain RGM deposit number 2529, uses of said formulation, and methods for stimulating the growth and/or increasing the yield of crops and/or protecting same from disease and pests, comprising said formulation.

Form PCT/ISA/210 (extra sheet) (January 2015)

INTERNATIONAL SEARCH REPORT
Information on patent family members

International application No.
PCT/CL2020/050169

| CN109456915 | 12-03-2019 | None | None |
| CN109468243A | 15-03-2019 | CN109456915B | 21-08-2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 109468243
- KR 1020190061210
- CN 109456915
- CN 108330092
- KR 1020140079201
- CN 108865934
- CN 106119146

### Non-patent literature cited in the description

- **MAYAK S. ; TIROSH T. ; GLICK B. R.** Plant growth promoting bacteria that confer resistance to water stress in tomato and pepper. *Plant Sci.,* 2004, vol. 166, 525-530
- **CRIOLLO P ; OBANDO M ; SANCHEZ L ; BONILLA R.** Effect of plant growth-promoting rhizobacteria (PGPR) associated to Pennisetum clandestinum in the highlands of Cundiboyacense. *Slurry Corpoica,* 2012, vol. 13 (2
- **ALTIER N ; BEYHAUT E ; DALLA M ; RIVAS F.** Environmental Sustainability: Platform for bio-inputs for agricultural use based on beneficial microorganisms. *INIA Uruguay Magazine,* 2012, vol. 29, 47-50
- **HAYAT R ; ALI S ; AMARA U ; KHALID R ; AHMED I.** Soil beneficial bacteria and their role in plant growth promotion: a review. *Ann Microbiol,* 2010, vol. 60 (4), 579-598
- **ETESAMI H ; EMAMI S ; ALIKHANI HA.** Potassium solubilizing bacteria (KSB): Mechanisms, promotion of plant growth, and future prospects - A review. *Journal of soil science and plant nutrition,* 2017, vol. 17, 897-911
- **YADAV S. ; KAUSHIK R. ; SAXENA A.K. ; ARORA D.K.** Genetic and functional diversity of Bacillus strains in the soils longterm irrigated with paper and pulp mill effluent. *J. Gen. Appl. Microbiol.,* 2011, vol. 57, 183-195
- **CHAKRABORTY U. ; CHAKRABORTY B.N. ; CHAKRABORTY A.P. ; DEY P.L.** Water stress amelioration and plant growth promotion in wheat plants by osmotic stress tolerant bacteria. *World J. Microbiol Biotechnol.,* 2013, vol. 29, 789-803
- **KAVAMURA V.N. ; SANTOS S.N. ; SILVA J.L. ; PARMA M.M. ; AVILA L.A. ; VISCONTI A. ; ZUCCHI T.D. ; TAKETANI R.G. ; ANDREOTE F.D. ; MELO I.S.** Screening of Brazilian cacti rhizobacteria for plant growth promotion under drought. *Microbiol. Res.,* 2013, vol. 168, 183-191
- **SUN H. ; HE Y. ; XIAO Q. ; YE R. ; TIAN Y.** Isolation, characterization, and antimicrobial activity of endophytic bacteria from Polygonum cuspidatum. *Afr. J. Microbiol. Res.,* 2013, vol. 7, 1496-1504
- **JEONG H ; JEONG DE ; KIM SH ; SONG GC ; PARK SY ; RYU CM ; PARK SH ; CHOI SK.** Draft Genome Sequence of the Plant Growth-Promoting Bacterium Bacillus siamensis KCTC 13613. *J Bacteriol,* 2012, vol. 194, 4148-4149
- **WU, L. ; WU, H. J. ; QIAO, J. ; GAO, X. ; BORRISS, R.** Novel routes for improving biocontrol activity of Bacillus based bioinoculants. *Front. Microbiol.,* 2015, vol. 6, 1395
- **HAEYOUNG JEONG ; DA-EUN JEONG ; SUN HONG KIM ; GEUN CHEOL SONG ; SOO-YOUNG PARK ; CHOONG-MIN RYU ; SEUNG-HWAN PARK AND SOO-KEUN CHOI.** Draft Genome Sequence of the Plant Growth-Promoting Bacterium Bacillus siamensis KCTC 13613. *J Bacteriol.,* 2012, vol. 194 (15), 4148-4149
- Use of plant-associated Bacillus strains as biofertilizers and biocontrol agents. **BORRISS, R.** Bacteria in Agrobiology: Plant Growth Response. Springer, 2011, 41-76
- The biology and potential biotechnological applications of Bacillus safensis. **AGBAJE LATEEF ; ISIAKA ADEDAYO ADELERE ; EVARISTE BOSCO GUEGUIM-KANA.** Biology. Section Cellular and Molecular Biology, 2015, vol. 70/4, 411-419